(19) Europäisches Patentamt
European Patent Office
Office européen des brevets

(11) EP 2 762 157 A1

(12) EUROPEAN PATENT APPLICATION

(43) Date of publication:
06.08.2014 Bulletin 2014/32

(51) Int Cl.:
*A61K 39/00* (2006.01) *A61K 39/12* (2006.01)

(21) Application number: 14000322.9

(22) Date of filing: 29.01.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 05.02.2013 JP 2013020731

(71) Applicant: NITTO DENKO CORPORATION
Ibaraki-shi, Osaka 567-8680 (JP)

(72) Inventors:
- Maeda, Yoshiki
Ibaraki-shi
Osaka 567-8680 (JP)
- Okubo, Katsuyuki
Ibaraki-shi
Osaka 567-8680 (JP)
- Asari, Daisuke
Ibaraki-shi
Osaka 567-8680 (JP)
- Okazaki, Arimichi
Ibaraki-shi
Osaka 567-8680 (JP)
- Shishido, Takuya
Ibaraki-shi
Osaka 567-8680 (JP)
- Matsushita, Kyohei
Ibaraki-shi
Osaka 567-8680 (JP)
- Li, Wenjing
Ibaraki-shi
Osaka 567-8680 (JP)
- Hori, Mitsuhiko
Ibaraki-shi
Osaka 567-8680 (JP)
- Sugiyama, Haruo
Suita-shi
Osaka 565-0871 (JP)

(74) Representative: Müller-Boré & Partner
Patentanwälte PartG mbB
Friedenheimer Brücke 21
80639 München (DE)

# (54) Vaccine composition for transdermal or mucosal administration

(57) The invention provides a vaccine composition for transdermal or transmucosal administration for inducing cellular immunity, comprising (i) an antigen; and (ii) a pharmacologically acceptable acid or a pharmacologically acceptable salt thereof as a first cellular immunity induction promoter.

EP 2 762 157 A1

SEQUENCE LISTING

<110> NITTO DENKO CORPORATION

<120> VACCINE COMPOSITION FOR TRANSDERMAL OR MUCOSAL ADMINISTRATION

<130> NITTO 2

<150> JP 2013-020731
<151> 2013-02-05

<160> 15

<170> PatentIn version 3.2

<210> 1
<211> 9
<212> PRT
<213> Homo sapiens

<400> 1

Ala Tyr Ala Cys Asn Thr Ser Thr Leu
1 5

<210> 2
<211> 9
<212> PRT
<213> Homo sapiens

<400> 2

Glu Tyr Ile Leu Ser Leu Glu Glu Leu
1 5

<210> 3
<211> 9
<212> PRT
<213> Homo sapiens

<400> 3

Thr Tyr Leu Pro Thr Asn Ala Ser Leu
1 5

<210> 4
<211> 9
<212> PRT
<213> Homo sapiens

<400> 4

Ile Met Pro Lys Ala Gly Leu Leu Ile
1 5

<210> 5
<211> 9
<212> PRT
<213> hepatitis C virus

<400> 5

Asp Leu Met Gly Tyr Ile Pro Ala Val
1 5

<210> 6
<211> 9
<212> PRT
<213> Homo sapiens

<400> 6

Val Leu Gln Glu Leu Asn Val Thr Val
1 5

<210> 7
<211> 9
<212> PRT
<213> Homo sapiens

<400> 7

Lys Val Phe Gly Ser Leu Ala Phe Val
1 5

<210> 8
<211> 9
<212> PRT
<213> Homo sapiens

<400> 8

Lys Val Ala Glu Ile Val His Phe Leu
1 5

<210> 9

<211> 9
<212> PRT
<213> hepatitis B virus

<400> 9

Trp Leu Ser Leu Leu Val Pro Phe Val
1               5

<210> 10
<211> 9
<212> PRT
<213> Homo sapiens

<400> 10

Lys Ile Phe Gly Ser Leu Ala Phe Leu
1               5

<210> 11
<211> 9
<212> PRT
<213> Homo sapiens

<400> 11

Ser Thr Ala Pro Pro Val His Asn Val
1               5

<210> 12
<211> 20
<212> DNA
<213> Unknown

<220>
<223> bacterial DNA

<400> 12
tccatgacgt tcctgacgtt 20

<210> 13
<211> 15
<212> PRT
<213> Mycobacterium tuberculosis

<400> 13

Phe Gln Asp Ala Tyr Asn Ala Ala Gly Gly His Asn Ala Val Phe

1 5 10 15

<210> 14
<211> 15
<212> PRT
<213> Artificial sequence

<220>
<223> Synthetic peptide

<400> 14

Phe Gln Asp Ala Tyr Asn Ala Val His Ala Ala His Ala Val Phe
1 5 10 15

<210> 15
<211> 13
<212> PRT
<213> Artificial sequence

<220>
<223> Synthetic peptide

<220>
<221> MOD_RES
<222> (1)..(1)
<223> D-alanine

<220>
<221> MOD_RES
<222> (3)..(3)
<223> cyclohexylalanine

<220>
<221> MOD_RES
<222> (13)..(13)
<223> D-alanine

<400> 15

Ala Lys Ala Val Ala Ala Trp Thr Leu Lys Ala Ala Ala
1 5 10

## Description

TECHNICAL FIELD

The present invention relates to vaccine compositions for transdermal or transmucosal administration.

BACKGROUND ART

A pathogen such as a microorganism or a virus, or a part of them is contained in a widely used vaccine and the vaccine is administered to induce immune response. In addition, there is a cancer vaccine which allows a cellular immune system to recognize an antigen specific for a cancer cell, and thereby induces an attack specific for the cancer cell by the immune system. The cancer vaccine has been used as one option for cancer therapy.

Usually, since invasion of the microorganism or virus is inhibited by the skin due to the size thereof, it is necessary that the vaccine is invasively administered into the body. Therefore, vaccines are usually administered by injection. However, the injection has some problems including pain, fear, injection scar, and subsequent scarring cicatrization. People other than health care workers are not permitted to perform the injection. Intradermal injection which can introduce higher immune response is a difficult administration technique. There is a risk of accidental infection of the health care workers due to needlestick injury. Patients are needed to visit a hospital repeatedly when administration is performed repetitively. Medical wastes which necessitate special disposition such as injection needles are generated. In view of the above issues, injection is not necessarily the optimal administration route.

The most popular administration route of vaccines is subcutaneous or intradermal injection; however, other immunity induction by various administration routes, for example, transdermal administration (Patent Document 1 and Non-Patent Document 1), buccal administration, nasal administration, or sublingual administration (Non-Patent Document 2, Patent Document 2, and Patent Document 3) has been attempted.

As an adjuvant which is generally used in immunization by an injection, aluminum salts, such as aluminium hydroxide, aluminium phosphate, or aluminum chloride; squalene containing emulsion, such as MF59 or AS03 are used in practice. In addition, components of flagellum, nucleic acids, cytokines, cation polymers, polypeptides, or the like have been widely examined. Adjuvants which has been examined in immunization by routes other than injection, for example, transdermal administration or transmucosal administration include aluminum salts, such as aluminium hydroxide, aluminium phosphate, or aluminum chloride; toxins, such as cholera toxin or heat-labile E. coli toxin; however, these adjuvants have never been used in practice. Most of them have been used as an adj uvant which induces a humoral immunity, the humoral immunity producing an antibody for preventing an infectious disease from a virus, a bacterium, and the like. On the other hand, as far as the cellular immunity induction is concerned, Freund's adjuvant, montanide, GM-CSF, IL-2, IL-12, or IFN-$\gamma$ has been examined for injections; however, they have not been used in practice. For transdermal administration or mucosal administration, only a few studies have been reported by using toxins, such as cholera toxin or heat-labile E. coli toxin, or nucleic acids.

LIST OF DOCUMENTS

[Patent Document 1] US Patent Application Publication No. US2008/0193487
[Patent Document 2] JP 2002-531415A
[Patent Document 3] US Patent Application Publication No. US2008/0112974
[Patent Document 4] JP H07-505883A
[Patent Document 5] JP 2007-529531A

[Non-Patent Document 1] Hosoi Akihiro et al., Cancer Research, 68, 3941-3949 (2008)
[Non-Patent Document 2] Zhengrong Cui et al., Pharmaceutical Research, Vol. 19, No. 7, 947-953 (2002)

SUMMARY OF THE INVENTION

Transdermal administration and transmucosal administration were attempted as a means for solving various problems associated with injection. However, no cellular immunity induction promoter which can be used effectively in the cellular immunity induction by transdermal or transmucosal administration of an antigen have been reported. Transdermal or transmucosal administration may have insufficient cellular immunity inducing effect, as compared with administration by injection.

Therefore, an object of the invention is to provide a vaccine composition having high convenience, and providing

high cellular immunity inducing effect in transdermal or transmucosal administration.

The inventors of the present invention researched substances suitable for cellular immunity induction by transdermal or transmucosal administration of an antigen. As a result, the inventors found that a pharmacologically acceptable acid or a pharmacologically acceptable salt thereof provides an effect of promoting cellular immunity induction. Further, the inventors found that cellular immunity induction is remarkably improved by using the acid or salt with at least one cellular immunity induction promoter selected from the group consisting of a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, and a Th2 cytokine inhibitor. Further, the inventors found that higher cellular immunity inducing effect is obtained by administering the vaccine formulation of the invention transdermally or transmucosally, as compared with administration by injections.

Further, in one aspect of the invention, when the vaccine composition of the invention is a formulation for transdermal administration, cellular immunity inducing effect can be further improved by administration under mildly irritating condition. Specifically, high cellular immunity inducing effect is obtained by administration of the vaccine composition for transdermal administration under a low stimulation condition in which before administration of the vaccine composition for transdermal administration, a model animal for evaluating skin irritation has a transepidermal water loss (TEWL) ($g/h \cdot m^2$) of 50 or less which is an index for skin. Alternatively, high cellular immunity inducing effect is obtained by selecting a low stimulation condition in which after administration of the vaccine composition for transdermal administration, a model animal for evaluating skin irritation has a cutaneous TSLP level (pg/mg protein) of 10,000 or less.

Therefore, the invention provides aspects as listed below:

(1) A vaccine composition for transdermal or transmucosal administration for inducing cellular immunity, comprising:

(i) an antigen; and
(ii) a pharmacologically acceptable acid or a pharmacologically acceptable salt thereof;

(2) The vaccine composition according to (1), wherein the pharmacologically acceptable acid or the pharmacologically acceptable salt thereof is an organic acid or a pharmacologically acceptable salt thereof;

(3) The vaccine composition according to (2), wherein the organic acid or the pharmacologically acceptable salt thereof is an organic compound containing carboxyl group or an organic compound containing sulfonate group, or a pharmacologically acceptable salt thereof;

(4) The vaccine composition according to (2), wherein the organic acid or the pharmacologically acceptable salt thereof is a saturated or unsaturated, linear or branched fatty acid with a saturated linear moiety having a carbon number of 8 to 20, lactic acid, malic acid, salicylic acid, maleic acid, citric acid, or an organic compound containing sulfonate group, or a pharmacologically acceptable salt thereof;

(5) The vaccine composition according to (2), wherein the organic acid or the pharmacologically acceptable salt thereof is a fatty acid selected from the group consisting of decanoic acid, lauric acid, myristic acid, isostearic acid, palmitic acid, stearic acid and oleic acid, or lactic acid, salicylic acid, citric acid or methanesulfonic acid, or a pharmacologically acceptable salt thereof;

(6) The vaccine composition according any one of (1)-(5), further comprising at least one cellular immunity induction promoter selected from the group consisting of a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, and a Th2 cytokine inhibitor;

(7) The vaccine composition according to (6), wherein the cellular immunity induction promoter is a TLR ligand;
(8) The vaccine composition according to (6), wherein the cellular immunity induction promoter is a cyclic dinucleotide;
(9) The vaccine composition according to (6), wherein the cellular immunity induction promoter is an immunomodulatory small molecule drug;
(10) The vaccine composition according to (6), wherein the cellular immunity induction promoter is a cyclooxygenase inhibitor;
(11) The vaccine composition according to (6), wherein the cellular immunity induction promoter is a prostaglandin receptor antagonist, and the prostaglandin receptor antagonist is an EP2 receptor antagonist, an EP4 receptor antagonist, a DP receptor antagonist, or an IP receptor antagonist;
(12) The vaccine composition according to (6), wherein the cellular immunity induction promoter is a prostaglandin receptor agonist, and the prostaglandin receptor agonist is an EP3 receptor agonist;
(13) The vaccine composition according to (6), wherein the cellular immunity induction promoter is a TSLP production inhibitor;
(14) The vaccine composition according to (6), wherein the cellular immunity induction promoter is an adenylate cyclase inhibitor;
(15) The vaccine composition according to (6), wherein the cellular immunity induction promoter is an omega-3 fatty acid;
(16) The vaccine composition according to (6), wherein the cellular immunity induction promoter is a PPAR agonist, and the PPAR agonist is a PPAR-$\alpha$ agonist, a PPAR-$\delta$ agonist, or a PPAR-$\gamma$ agonist;
(17) The vaccine composition according to (6), wherein the cellular immunity induction promoter is a dopamine receptor antagonist, and the dopamine receptor antagonist is a D1 receptor antagonist, or a D5 receptor antagonist;
(18) The vaccine composition according to (6), wherein the cellular immunity induction promoter is a dopamine receptor agonist, and the dopamine receptor agonist is a D2 receptor agonist, a D3 receptor agonist, or a D4 receptor agonist;
(19) The vaccine composition according to (6), wherein the cellular immunity induction promoter is a histamine receptor antagonist, and the histamine receptor antagonist is an H1 receptor antagonist, or an H2 receptor antagonist;
(20) The vaccine composition according to (6), wherein the cellular immunity induction promoter is a histamine receptor agonist, and the histamine receptor agonist is an H1 receptor agonist, an H3 receptor agonist, or an H4 receptor agonist;
(21) The vaccine composition according to (6), wherein the cellular immunity induction promoter is a serotonin receptor antagonist, and the serotonin receptor antagonist is a 5-HT2 receptor antagonist, a 5-HT4 receptor antagonist, a 5-HT6 receptor antagonist, or a 5-HT7 receptor antagonist;
(22) The vaccine composition according to (6), wherein the cellular immunity induction promoter is a serotonin receptor agonist, and the serotonin receptor agonist is a 5-HT1 receptor agonist, or a 5-HT2 receptor agonist;
(23) The vaccine composition according to (6), wherein the cellular immunity induction promoter is a vasopressin receptor antagonist, and the vasopressin receptor antagonist is a V2 receptor antagonist;
(24) The vaccine composition according to (6), wherein the cellular immunity induction promoter is a vasopressin receptor agonist, and the vasopressin receptor agonist is a V1 receptor agonist;
(25) The vaccine composition according to (6), wherein the cellular immunity induction promoter is a muscarine receptor antagonist, and the muscarine receptor antagonist is an M1 receptor antagonist, an M3 receptor antagonist, and an M5 receptor antagonist;
(26) The vaccine composition according to (6), wherein the cellular immunity induction promoter is a muscarine receptor agonist, and the muscarine receptor agonist is an M1 receptor agonist, an M2 receptor agonist, an M3 receptor agonist, an M4 receptor agonist, or an M5 receptor agonist;
(27) The vaccine composition according to (6), wherein the cellular immunity induction promoter is an adrenergic receptor antagonist, and the adrenergic receptor antagonist is an $\alpha$1 receptor antagonist, a $\beta$1 receptor antagonist, a $\beta$2 receptor antagonist, or a $\beta$3 receptor antagonist;
(28) The vaccine composition according to (6), wherein the cellular immunity induction promoter is an adrenergic receptor agonist, and the adrenergic receptor agonist is an $\alpha$1 receptor agonist, and an $\alpha$2 receptor agonist;
(29) The vaccine composition according to (6), wherein the cellular immunity induction promoter is an angiotensin receptor agonist, and the angiotensin receptor agonist is an AT2 receptor agonist;
(30) The vaccine composition according to claim 1, wherein the cellular immunity induction promoter is a GABA receptor agonist, and the GABA receptor agonist is a $GABA_B$ receptor agonist;
(31) The vaccine composition according to (6), wherein the cellular immunity induction promoter is a thrombin receptor antagonist, and the thrombin receptor antagonist is a PAR-1 receptor antagonist;
(32) The vaccine composition according to (6), wherein the cellular immunity induction promoter is a thrombin receptor agonist, and the thrombin receptor agonist is a PAR-1 receptor agonist;
(33) The vaccine composition according to (6), wherein the cellular immunity induction promoter is an opioid receptor

agonist;
(34) The vaccine composition according to (6), wherein the cellular immunity induction promoter is a leukotriene receptor antagonist, and the leukotriene receptor antagonist is a CysLT1 receptor antagonist, and a CysLT2 receptor antagonist;
(35) The vaccine composition according to (6), wherein the cellular immunity induction promoter is a leukotriene receptor agonist, and the leukotriene receptor agonist is a BLT receptor agonist.
(36) The vaccine composition according to (6), wherein the cellular immunity induction promoter is a melatonin receptor agonist.
(37) The vaccine composition according to (6), wherein the cellular immunity induction promoter is a somatostatin receptor agonist.
(38) The vaccine composition according to (6), wherein the cellular immunity induction promoter is a cannabinoid receptor agonist.
(39) The vaccine composition according to (6), wherein the cellular immunity induction promoter is a sphingosine-1 phosphate receptor agonist.
(40) The vaccine composition according to (6), wherein the cellular immunity induction promoter is a metabotropic glutamate receptor agonist, and the metabotropic glutamate receptor agonist is an mGluR2 receptor agonist, an mGluR3 receptor agonist, an mGluR4 receptor agonist, an mGluR6 receptor agonist, an mGluR7 receptor agonist, or an mGluR8 receptor agonist.
(41) The vaccine composition according to (6), wherein the cellular immunity induction promoter is an ADP receptor agonist;
(42) The vaccine composition according to (6), wherein the cellular immunity induction promoter is a phospholipase A2 inhibitor;
(43) The vaccine composition according to (6), wherein the cellular immunity induction promoter is a TGF-β production inhibitor;
(44) The vaccine composition according to (6), wherein the cellular immunity induction promoter is a Th2 cytokine inhibitor;
(45) The vaccine composition according to (6), wherein the cellular immunity induction promoter is a helper peptide;
(46) The vaccine composition according to (6), wherein the cellular immunity induction promoter is a combination of at least one selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor, and a helper peptide;
(47) The vaccine composition according to any one of (1) - (46), wherein the antigen is a peptide selected from the group consisting of survivin-2B peptide and/or a modified survivin-2B. peptide, GPC3 peptide and/or a modified GPC3 peptide, HER2/neu_A24 peptide and/or a modified HER2/neu_A24 peptide, MAGE3_A24 peptide and/or a modified MAGE3_A24 peptide, IPEP87 peptide and/or a modified IPEP87 peptide, PR1 peptide and/or a modified PR1 peptide, HER2/neu_A02 peptide and/or a modified HER2/neu_A02 peptide, MAGE3_A02 peptide and/or a modified MAGE3_A02 peptide, HBVenv peptide and/or a modified HBVenv peptide, and a peptide selected from the group consisting of MUC1 peptide and/or a modified MUC1 peptide;
(48) The vaccine composition for transdermal administration according to any one of (1)-(47), wherein the composition is administered under mildly irritating condition;
(49) The vaccine composition for transdermal administration according to (48), wherein the mildly irritating condition is one in which transepidermal water loss (TEWL) of model animals for evaluating skin irritation before administration is 50 $g/h \cdot m^2$ or less; and
(50) The vaccine composition for transdermal administration according to (48) or (49), wherein the mildly irritating condition is one in which TSLP level in the skin of model animals for evaluating skin irritation after administration is 10,000 pg/mg protein or less.

In another aspect, the vaccine composition of the invention can be used for treating or preventing a disease. Therefore, the invention also provides aspects as listed below:

(51) A method for treating or preventing a cancer, comprising administrating a therapeutically effective amount of (i) a cancer antigen, and (ii) a pharmacologically acceptable acid or a pharmacologically acceptable salt thereof to a subject transdermally or transmucosally;
(52) The method according to (51), wherein the cancer antigen is a cancer antigen peptide selected from the group consisting of survivin-2B peptide and/or a modified survivin-2B peptide, GPC3 peptide and/or a modified GPC3 peptide, HER2/neu_A24 peptide and/or a modified HER2/neu_A24 peptide, MAGE3_A24 peptide and/or a modified MAGE3_A24 peptide, PR1 peptide and/or a modified PR1 peptide, HER2/neu_A02 peptide and/or a modified HER2/neu_A02 peptide, MAGE3_A02 peptide and/or a modified MAGE3_A02 peptide, and a peptide selected from the group consisting of MUC1 peptide and/or a modified MUC1 peptide;
(53) A method for treating or preventing a viral disease, comprising administrating a therapeutically effective amount of (i) a virus antigen, and (ii) a pharmacologically acceptable acid or a pharmacologically acceptable salt thereof to a subject transdermally or transmucosally; and
(54) The method according to (53), wherein the virus antigen is a peptide selected from the group consisting of IPEP87 peptide and/or a modified IPEP87 peptide, and a peptide selected from the group consisting of HBVenv peptide and/or a modified HBVenv peptide.

In another aspect, the invention also provides a pharmacologically acceptable acid or a pharmacologically acceptable salt thereof for use as a cellular immune activator for transdermal or transmucosal administration of an antigen. Therefore, the invention also provides the following aspects:

(55) A pharmacologically acceptable acid or a pharmacologically acceptable salt thereof for use as a cellular immune activator for immunity by transdermal or transmucosal administration; and
(56) A combination of (i) pharmacologically acceptable acid or a pharmacologically acceptable salt thereof, and (ii) at least one substance selected from the group consisting of a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-$\beta$ production inhibitor, and a Th2 cytokine inhibitor, for use as a cellular immune activator for immunity by transdermal or transmucosal administration.

The present invention also provides the following embodiments:

(57) A method of inducing cellular immunity, which comprises transdermally or transmucosally administering to a subject (i) an antigen and (ii) a pharmacologically acceptable acid or a pharmacologically acceptable salt thereof;
(58) A pharmacologically acceptable acid or a pharmacologically acceptable salt thereof, for use in stimulating cellular immunity by the transdermal or transmucosal administration of an antigen;
(59) A combination of (i) a pharmacologically acceptable acid or a pharmacologically acceptable salt thereof and (ii) one or more cellular immunity induction promoters selected from the group consisting of TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor and Th2 cytokine inhibitor, for use in stimulating cellular immunity by the transdermal or transmucosal administration of an antigen;
(60) A combination of (i) an antigen and (ii) a pharmacologically acceptable acid or a pharmacologically acceptable salt thereof for use in inducing cellular immunity against an antigen, wherein the combination is transdermally or transmucosally administered;

(61) A combination of (i) an antigen, (ii) a pharmacologically acceptable acid or a pharmacologically acceptable salt thereof, and (iii) one or more cellular immunity induction promoters selected from the group consisting of TLR ligand, cyclic dinucleotide, helper peptide, immunomodulatory small molecule drug, cyclooxygenase inhibitor, prostaglandin receptor antagonist, prostaglandin receptor agonist, TSLP production inhibitor, adenylate cyclase inhibitor, omega-3 fatty acid, PPAR agonist, dopamine receptor antagonist, dopamine receptor agonist, histamine receptor agonist, histamine receptor antagonist, serotonin receptor agonist, serotonin receptor antagonist, vasopressin receptor antagonist, vasopressin receptor agonist, muscarine receptor antagonist, muscarine receptor agonist, adrenalin receptor antagonist, adrenalin receptor agonist, angiotensin receptor agonist, GABA receptor agonist, thrombin receptor antagonist, thrombin receptor agonist, opioid receptor agonist, ADP receptor agonist, leukotriene receptor antagonist, leukotriene receptor agonist, melatonin receptor agonist, somatostatin receptor agonist, cannabinoid receptor agonist, sphingosine-1 phosphate receptor agonist, metabotropic glutamate receptor agonist, phospholipase A2 inhibitor, TGF-beta production inhibitor and Th2 cytokine inhibitor, for use in inducing cellular immunity against an antigen, wherein the combination is transdermally or transmucosally administered;

(62) A combination of (i) a cancer antigen and (ii) a pharmacologically acceptable acid or a pharmacologically acceptable salt thereof for use in treating or preventing a cancer, wherein the combination is transdermally or transmucosally administered;

(63) A combination of (i) a virus antigen and (ii) a pharmacologically acceptable acid or a pharmacologically acceptable salt thereof for use in treating or preventing a viral disease, wherein the combination is transdermally or transmucosally administered;

(64) Use of (i) an antigen and (ii) a pharmacologically acceptable acid or a pharmacologically acceptable salt thereof for the manufacture of vaccine composition for transdermal or transmucosal administration intended for the induction of cellular immunity;

(65) Use of (i) a cancer antigen and (ii) a pharmacologically acceptable acid or a pharmacologically acceptable salt thereof for the manufacture of vaccine composition for transdermal or transmucosal administration for the treatment or prevention of a cancer; and

(66) Use of (i) a virus antigen and (ii) a pharmacologically acceptable acid or a pharmacologically acceptable salt thereof for the manufacture of vaccine composition for transdermal or transmucosal administration for the treatment or prevention of a viral disease.

The vaccine composition of the invention can be administered transdermally or transmucosally (in particular, buccal administration including sublingual mucous membrane). Therefore, the vaccine composition of the invention has advantages that it increases compliance, for example, it can be administered non-invasively, painlessly, or with no fear of injection, patients can administer it to themselves because the administration is convenient, health care workers can avoid a risk of a needlestick injury, the burden of going to a hospital can be decreased if it is repeatedly administered and thereby patients' quality of life can be improved, it produces no medical wastes which needs to be discarded in a special way (for example, needles for injection). Further, in the case of patches such as cataplasm preparations or tape preparations, the composition has advantages that a predetermined dosage can be administered surely, a drug-release rate can be suitably controlled, and it doesn't attach undesired sites when administered. Further, since patches can be easily attached and removed, if patients experience any side effect, the composition has advantages that patients can stop the administration immediately by removing the patch from the site of administration. Further, the vaccine composition of the invention has an advantage that the efficacy is remarkably improved as compared with the administration of an antigen only. Further, the vaccine composition of the invention has an advantage that the composition can induce stronger immunity than administration by injection.

## DETAILED DESCRIPTION OF THE INVENTION

The terms as used herein are defined as follows for the purpose of easier understanding of the invention. The terms having no definition herein have the meanings generally understood by a person skilled in the art, in particular, in medical, pharmaceutical, immunology, cell biology, biochemistry, polymer chemistry area, or the like, unless otherwise indicated by the context.

### I. definitions

As used herein, the term "antigen" means all substances being capable of inducing immune response, including proteins, and peptides. For transdermal or transmucosal administration which needs a skin permeability and mucosa permeability of an antigen, a low molecular weight antigen is preferably used, for example, a peptide having about 8 to about 12 amino acids can be used. In the invention, for example, survivin-2B peptide, GPC3 peptide, HER2/neu_A24 peptide, MAGE3_A24 peptide, IPEP87 peptide, PR1 peptide, HER2/neu_A02 peptide, MAGE3_A02 peptide, HBVenv

peptide, HER2/neu E75 peptide, and MUC1 peptide as described below can be used as the antigen. In one embodiment, the antigen used in the vaccine composition of the invention excludes at least one peptide selected from the group consisting of WT1 peptide for cancer vaccine, and a modified WT1 peptide for cancer vaccine. In one embodiment, one or more peptides selected from the group consisting of a_HER2/neu E75 peptide for use as a cancer vaccine and a modified HER2/neu E75 peptide for use as a cancer vaccine are excluded from the antigen used in the vaccine composition of the present invention.

As used herein, the term "survivin-2B peptide" means a peptide derived from a cancer gene product survivin, the peptide consisting of a sequence Ala Tyr Ala Cys Asn Thr Ser Thr Leu (SEQ ID NO: 1).

As used herein, the term "GPC3 peptide" means a peptide derived from a cancer gene product GPC3, the peptide consisting of a sequence Glu Tyr Ile Leu Ser Leu Glu Leu (SEQ ID NO: 2).

As used herein, the term "HER2/neu_A24 peptide" means a HLA-A24-binding peptide derived from a cancer gene product HER2/neu, the peptide consisting of a sequence Thr Tyr Leu Pro Thr Asn Ala Ser Leu (SEQ ID NO: 3).

As used herein, the term "MAGE3_A24 peptide" means a HLA-A24-binding peptide derived from a cancer gene product MAGE3, the peptide consisting of a sequence Ile Met Pro Lys Ala Gly Leu Ile (SEQ ID NO: 4).

As used herein, the term "IPEP87 peptide" means a peptide derived from a hepatitis C virus (HCV) protein, the peptide consisting of a sequence Asp Leu Met Gly Tyr Ile Pro Ala Val (SEQ ID NO: 5).

As used herein, the term "PR1 peptide" means a peptide derived from a cancer gene product proteinase-3, the peptide consisting of a sequence Val Leu Gln Glu Leu Asn Val Thr Val (SEQ ID NO: 6).

As used herein, the term "HER2/neu_A02 peptide" means a HLA-A02 restricted peptide derived from a cancer gene product HER2/neu, the peptide consisting of a sequence Lys Val Phe Gly Ser Leu Ala Phe Val (SEQ ID NO: 7).

As used herein, the term "MAGE3_A02 peptide" means a HLA-A02 restricted peptide derived from a cancer gene product MAGE3, the peptide consisting of a sequence Lys Val Ala Glu Ile Val His Phe Leu (SEQ ID NO: 8).

As used herein, the term "HBVenv peptide" means a peptide derived from a Hepatitis B virus (HBV) protein, the peptide consisting of a sequence Trp Leu Ser Leu Val Pro Phe Val (SEQ ID NO: 9).

As used herein, the term "HER2/neu E75 peptide" means a peptide derived from a cancer gene HER2/neu product (HER2 protein), the peptide consisting of a sequence Lys Ile Phe Gly Ser Leu Ala Phe Leu (SEQ ID NO: 10).

As used herein, the term "MUC1 peptide" means a peptide derived from a glycoprotein MUC1 protein highly expressing in many cancer cells, the peptide consisting of a sequence Ser Thr Ala Pro Val His Asn Val (SEQ ID NO: 11).

As used herein, the term "WT1 peptide" means a partial peptide having about 8-15 amino acids, preferably about 8-12 amino acids which is a fragment of the product of the WT1 oncogene (Wilm's tumor), WT1 protein, including a Db126 peptide or a Db235 peptide (both are described in Patent No. 4422903). Further, the partial peptide of the WT1 product as disclosed in WO2000/06602, the WT1 derived HLA-A26-binding cancer antigen peptide as described in WO2005/095598, the HLA-A*3303-restricted WT1 peptide as described in WO2007/097358, and the HLA-A*1101-restricted WT1 peptide as described in WO2008/081701 are also included in the "WT1 peptide" as described herein.

As used herein, the term "modified XX peptide" (XX is a name of any peptide) means a peptide modified by substitution, modification, or the like of all or some amino acids of the XX peptide. Modified XX peptide includes, for example,

(a) a peptide consisting of an amino acid sequence in which one or more, for example, one, two, three, four, or five amino acids are substituted, deleted, or added in the amino acid sequence of the XX peptide; and

(b) a peptide consisting of an amino acid sequence in which all or some amino acids, for example, one or more, for example, one, two, three, four, five, six, seven, eight, nine, or ten amino acids are modified in the amino acid sequence of the XX peptide.

The "modification" of amino acids in which the modified XX peptide can have includes, but not limited to, for example, acetylation, alkylation such as methylation, glycosylation, hydroxylation, carboxylation, aldehyde formation, phosphorylation, sulfonylation, formylation, modification by aliphatic chain addition such as myristoylation, palmitoylation, or stearoylation, octanolyation, esterification, amidation, deamidation, modification by disulfide bond formation such as cystine modification, glutathione modification, or thioglycolic acid modification, glycosylation, ubiquitination, succinimide formation, glutamylation, and prenylation. The modified XX peptide may include a combination of a substitution, deletion, or addition of at least one amino acid, and a modification of at least one amino acid.

In a preferred aspect, an antigen included in the vaccine composition of the invention for transdermal administration is a peptide selected from the group consisting of survivin-2B peptide and/or a modified survivin-2B peptide, GPC3 peptide and/or a modified GPC3 peptide, HER2/neu_A24 peptide and/or a modified HER2/neu_A24 peptide, MAGE3_A24 peptide and/or a modified MAGE3_A24 peptide, IPEP87 peptide and/or a modified IPEP87 peptide, PR1 peptide and/or a modified PR1 peptide, HER2/neu_A02 peptide and/or a modified HER2/neu_A02 peptide, MAGE3_A02 peptide and/or a modified MAGE3_A02 peptide, HBVenv peptide and/or a modified HBVenv peptide, and MUC1 peptide and/or a modified MUC1 peptide. Alternatively, HER2/neu E75 peptide and/or a modified HER2/neu E75 peptide may

be used as an antigen.

The peptide as listed above may be a free form or the form of any pharmacologically acceptable salt, for example, as acid salt (such as, acetate, TFA salt, hydrochloride, sulfate, phosphate, lactate, tartrate, maleate, fumarate, oxalate, hydrobromate, succinate, nitrate, malate, citrate, oleate, palmitate, propionate, formate, benzoate, picrate, benzenesulfonate, dodecylsulfate, methanesulfonate, p-toluenesulfonate, glutarate, or various amino acid salts), a metal salt (alkali metal salt (for example, sodium salt, or potassium salt), alkaline earth metal salt (for example, calcium salt, or magnesium salt), aluminum salt, or the like), amine salt (triethylamine salt, benzylamine salt, diethanol amine salt, t-butylamine salt, dicyclohexylamine salt, alginine salt, dimethyl ammonium salt, ammonium salt, or the like). Preferred pharmacologically acceptable salts are acetate salts or TFA salts. The peptide which can be used as an antigen in the invention can be used in a synthesized or produced, isolated and purified form by any well-known method.

As used herein, the term "cellular immunity induction promoter" means any substance which can improve the efficiency of cellular immunity of the antigen administered together with the substance as compared with that in absence of the substance. The cellular immunity induction promoter is not limited by the mechanism of promoting cellular immunity induction, and means one as defined herein.

As used herein, "pharmacologically acceptable acid" which can be included in the pharmaceutical composition of the invention as the first cellular immunity induction promoter means acids without providing adverse action to the administered subject and without interfering with the pharmacological activity of the components in the pharmaceutical composition. In a preferred aspect of the invention, the pharmacologically acceptable acid is an organic acid, more preferably an organic compound containing carboxyl group or an organic compound containing sulfonate group, more preferably, a saturated or unsaturated, linear or branched fatty acid with a saturated linear moiety having a carbon number of 8 to 20, lactic acid, malic acid, salicylic acid, maleic acid, citric acid, or an organic compound containing sulfonate group, more preferably, a saturated or unsaturated, linear or branched fatty acid with a saturated linear moiety having a carbon number of 8 to 16, lactic acid, malic acid, salicylic acid, maleic acid, citric acid, or an organic compound containing sulfonate group, still more preferably, a fatty acid selected from the group consisting of decanoic acid, lauric acid, myristic acid, isostearic acid, palmitic acid, stearic acid and oleic acid, or lactic acid, salicylic acid, citric acid or methanesulfonic acid.

As used herein, "pharmacologically acceptable salt" which can be included in the pharmaceutical composition of the invention means salts without providing adverse action to the administered subject and without interfering with the pharmacological activity of the components in the pharmaceutical composition. The pharmacologically acceptable salt includes inorganic acid salts (for example, hydrochloride or phosphate), organic acid salts (for example, acetate or phthalate, TFA acid salt), metal salts (alkali metal salts (for example, sodium salts, or potassium salts), alkaline earth metal salts (for example, calcium salts, or magnesium salts), aluminum salts, or the like), amine salts (triethylamine salts, benzylamine salts, diethanol amine salts, t-butylamine salts, dicyclohexylamine salts, alginine salts, dimethyl ammonium salts, ammonium salts, or the like), without limitation.

As used herein, the term "TLR ligand" means a ligand for a Toll-like receptor (TLR), for example, including a ligand for TLR1-9. The TLR ligand includes a ligand for a heterodimer of TLR1 and TLR2 (a TLR1/2 ligand), a ligand for a heterodimer of TLR2 and TLR6 (a TLR2/6 ligand), a ligand for TLR2 and Dectin1, a TLR3 ligand, a TLR4 ligand, a TLR5 ligand, a TLR7 ligand and/or a TLR8 ligand, and a TLR9 ligand, all of which can be used as the cellular immunity induction promoter of the invention. In a preferred aspect of the invention, the TLR ligand is a TLR1/2 ligand, a TLR2 and Dectin1 ligand, a TLR3 ligand, a TLR4 ligand, a TLR7 ligand and/or a TLR8 ligand, and/or a TLR9 ligand.

As used herein, the term "TLR1/2 ligand" means a ligand for a heterodimer of a Toll-like receptor (TLR) 1 and a Toll-like receptor (TLR) 2, for example, triacylated lipoprotein derived from bacteria cell walls and salts thereof, they may be an extract, a product, or a synthetic compound thereof, without limitation.

In a preferred aspect of the invention, the TLR1/2 ligand is $Pam_3CSK_4$. $Pam_3CSK_4$ has the formula:

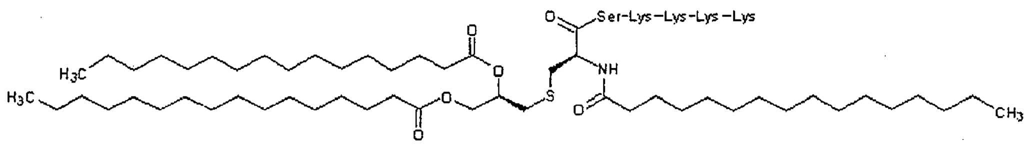

As used herein, the term "TLR2 and Dectin1 ligand" means a ligand for a Toll-like receptor (TLR) 2 and a β1,3-glucan receptor (Dectin1), for example, including a β1,3-glucan derived from cell walls of fungus and salts thereof. They may be an extract, a product, or a synthetic compound thereof, without limitation. In a preferred aspect of the invention, the TLR2 and Dectin1 ligand is Zymosan derived from cell walls of yeast.

As used herein, the term "TLR3 ligand" means a ligand for a Toll-like receptor (TLR) 3, for example, including a double-stranded RNA (dsRNA) derived from virus and salts thereof. They may be an extract, a product, or a synthetic compound thereof, without limitation. In a preferred aspect of the invention, the TLR3 ligand is a synthetic compound

polyinosinic-polycytidylic acid (Poly (I:C)) and/or salts thereof.

As used herein, the term "TLR4 ligand" means a ligand for a Toll-like receptor (TLR) 4, for example, a lipopolysaccharide (LPS) derived from bacteria or plants, in particular, lipid A derivatives, for example, monophosphoryl lipid A, 3 deacylated monophosphoryl lipid A (3D-MPL), OM174, OM 294 DP, or OM 197 MP-Ac DP, alkyl glucosaminide phosphate (AGP), for example, AGP as disclosed in WO 9850399 or US 6303347 or AGP salt as disclosed in US 6764840. Further, the ligand includes lipopolysaccharide derived from Pantoea, glucopyranosyl lipid, sodium hyaluronate, without limitation.

In a preferred aspect of the invention, the TLR4 ligand is preferably a lipopolysaccharide derived from Acetobacter genus (for example, Acetobacter aceti, Acetobacter xylinum, Acetobacter orientalis), Zymomonas genus (for example, Zymomonas mobilis), Xanthomonas genus (for example, Xanthomonas campestris), Enterobacter genus (for example, Enterobacter cloacae), Pantoea genus (for example, Pantoea agglomerans). An extract of the lipopolysaccharide or purified lipopolysaccharide can be used as such. Further, for example, the lipopolysaccharide derived from Pantoea agglomerans (IP-PA1) can be commercially available from Funakoshi Co., Ltd. Further, in a preferred aspect of the invention, the TLR4 ligand is a lipopolysaccharide derived from Pantoea, a glucopyranosyl lipid, and/or a sodium hyaluronate.

As used herein, the term "TLR7 and/or TLR8 ligand" means a ligand for a Toll-like receptor (TLR) 7 and/or TLR8, for example, including a single-stranded RNA, imiquimod, resiquimod (R848), TLR7-II, and other compounds, for example, loxoribine and bropirimine, without limitation.

In a preferred aspect of the invention, the TLR7 ligand and/or the TLR8 ligand is imiquimod. Imiquimod is 1-(2-methylpropyl)-1H-imidazo[4,5-c]quinoline-4-amine having the formula:

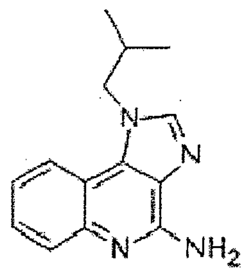

For example, JP H07-505883A (Patent Document 4) describes the characteristics and a method for producing it.

In another preferred aspect, the TLR7 ligand and/or the TLR8 ligand is resiquimod. Resiquimod is 4-amino-2-(ethoxymethyl)-α,α-dimethyl-1H-imidazo[4,5-c]quinoline-1-et hanol having the formula:

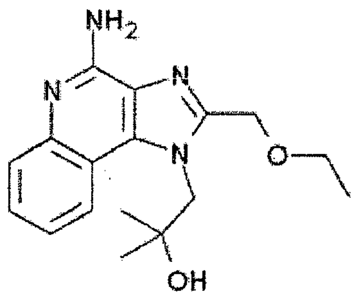

In another preferred aspect, the TLR7 ligand and/or the TLR8 ligand is TLR7-II. TLR7-II is represented by the formula:

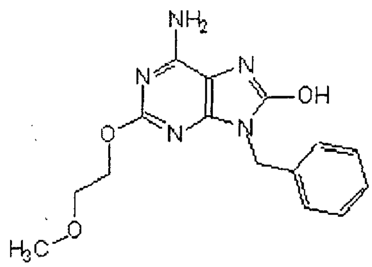

In another preferred aspect, the TLR7 ligand and/or the TLR8 ligand is bropirimine. Bropirimine is represented by the formula:

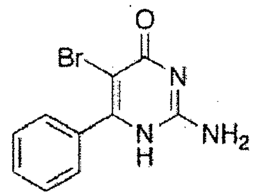

As used herein, the term "TLR9 ligand" means a ligand for a Toll-like receptor (TLR) 9, for example, including ODN1826. The TLR9 ligand used in the invention may be an extract, a product, or a synthetic compound thereof, without limitation. In a preferred aspect of the invention, TLR9 ligand is ODN1826.

ODN1826 is an oligodeoxynucleotide having the following sequence (SEQ ID NO: 12):
5'-tccatgacgttcctgacgtt-3'

As used herein, the term "TLR2/6 ligand" means a ligand of a heterodimer for a Toll-like receptor (TLR) 2 and a Toll-like receptor (TLR) 6, for example, including a diacylated lipoprotein derived from cell walls of mycoplasma and salts thereof. They may be an extract, a product, or a synthetic compound thereof, without limitation. In a preferred aspect of the invention, the TLR2/6 ligand is $Pam_2$ $CSK_4$, MALP-2 and/or FSL-1.

$Pam_2CSK_4$ is represented by the following formula:

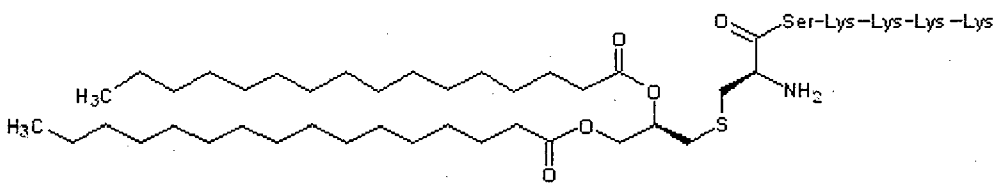

FSL-1 is represented by the following formula:

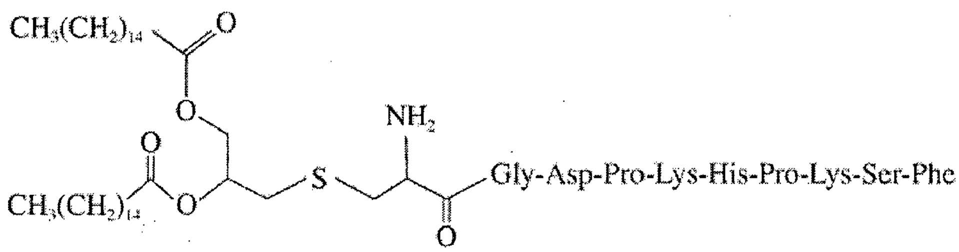

As used herein, the term "TLR5 ligand" means a ligand for a Toll-like receptor (TLR) 5, for example, including flagellin. The TLR5 ligand used in the invention may be an extract, a product, or a synthetic compound thereof, without limitation. In a preferred aspect of the invention, TLR5 ligand is flagellin.

The Toll-like receptor (TLR) is a family of I-type transmembrane protein in which the in vivo activation initiates an innate immune response involving a specific cytokine, a chemokine and a growth factor. All TLR can activate a certain intracellular signaling molecule, for example, nuclear factor κB (NF-κB) and mitogen-activated protein kinase (MAP kinase), while the specific population of the released cytokine and chemokine is likely to be unique for each TLR. The TLR3, 7, 8, and 9 include a subfamily of TLR presented in the endosome area or the lysosome area of immune cells (dendritic cells and monocytes). Specifically, the TLR3 is expressed by various cells including dendritic cells or fibroblasts. The TLR7 is expressed by plasmacytoid dendritic cells, and expressed by monocytes in relatively small level. The TLR8 is expressed by monocytes and monocyte derived dendritic cells and myeloid dendritic cells. The TLR9 is expressed by plasmacytoid dendritic cells. The subfamily mediates the recognition of a microorganism nucleic acid (such as a single-stranded RNA, a double-stranded RNA, or a single-stranded DNA). The TLR3, TLR7 and/or TLR8, TLR9 agonist stimulates the production of various inflammatory cytokines (for example, including interleukin-6, interleukin-12, TNF-α, and interferon-γ). The agonist further promotes increase in the expression of a co-stimulatory molecule (for example, CD40, CD80, and CD86), a major histocompatibility complex molecule, and a chemokine receptor. An I-type interferon (IFNα and IFNβ) is further produced by cells during the activation of the TLR7 and/or TLR8 agonist.

As used herein, the term "cyclic dinucleotide" means a cyclized molecule in which each of 2 OH groups in the

saccharide moieties of two nucleotides forms an ester with the same phosphate molecule, and analogs thereof, for example, including a cyclic diAMP (c-di-AMP), a cyclic diGMP (c-di-GMP), a c-dGpGp, a c-dGpdGp, a c-GpAp, a c-GpCp, and a c-GpUp, without limitation. The cyclic dinucleotide activates dendritic cells or T cells. Further examples of the cyclic dinucleotide, the usability of the cyclic dinucleotide as an adjuvant, and a method for producing thereof are described in JP 2007-529531A (Patent Document 5). In a preferred aspect of the invention, the cyclic dinucleotide is a cyclic diGMP and/or a cyclic diAMP. The cyclic diGMP has the formula.

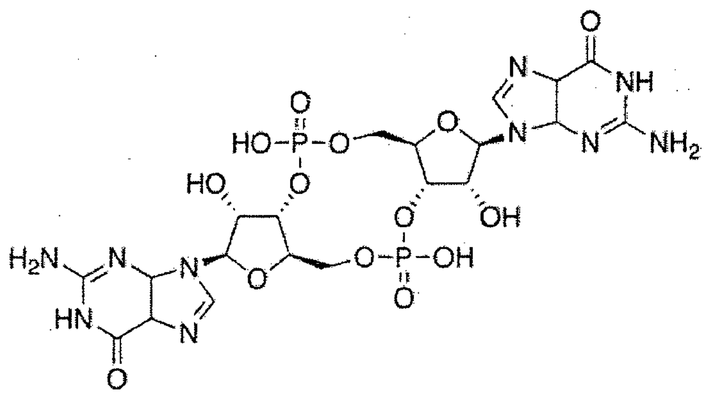

The synthesis method is described in Kawai et al., Nucleic Acids Research Suppl.3: 103-4.

As used herein, the term "helper peptide" means any peptide which activates helper T cells, for example, including a helper peptide derived from tuberculosis, a helper peptide derived from measles virus, a helper peptide derived from Hepatitis B virus, a helper peptide derived from hepatitis C virus, a helper peptide derived from Chlamydia trachomatis, a helper peptide derived from P. falciparum sporozoite, a helper peptide derived from keyhole limpet haemocyanin, a helper peptide derived from tetanus toxin, a helper peptide derived from pertussis toxin, a helper peptide derived from diphtheria toxin, a helper peptide derived from cancer cell (for example, an IMA-MMP-001 helper peptide, a CEA-006 helper peptide, an MMP-001 helper peptide, a TGFBI-004 helper peptide, an HER-2/neu (aa776-790) helper peptide, an AE36 helper peptide, an AE37 helper peptide, an MET-005 helper peptide, a BIR-002 helper peptide), and universal helper analogs (for example, PADRE). In a preferable aspect of the present invention, the helper peptide consists of 10 to 18 amino acids, preferably 12 to 16 amino acids, more preferably 13 to 15 amino acids.

In a preferred aspect of the invention, the helper peptide is Peptide-25 or a modified Peptide-25 or PADRE. An example of the modified Peptide-25 is Peptide-25B. Peptide-25 is a peptide of 15 amino acids consisting of a sequence Phe Gln Asp Ala Tyr Asn Ala Gly His Asn Ala Val Phe (SEQ ID NO: 13), the sequence corresponds to the amino acid residue 240-254 of Ag85B, which is a major protein secreted by human-type tuberculosis (Mycobacterium tuberculosis). Peptide-25B is an example of the modified Peptide-25 in which the part of amino acids of Peptide-25 is modified in order to improve the cellular immunity inducing effect, and a peptide of 15 amino acids consisting of a sequence Phe Gln Asp Ala Tyr Asn Ala Val His Ala His Ala Val Phe (SEQ ID NO: 14). PADRE is a peptide of 13 amino acids consisting of a sequence D-Ala Lys cyclohexyl-Ala Val Ala Ala Trp Thr Leu Lys Ala Ala D-Ala (Herein referred as SEQ ID NO: 15).

Further, in the invention, instead of the helper peptide, or in combination with the helper peptide, any peptide modified by substitution or modification of all or some amino acids of the helper peptide (hereinafter referred as "modified helper peptide") can also be used. The modified helper peptide includes, for example,

(a) a peptide consisting of an amino acid sequence in which one or more, for example, one, two, three, four, or five amino acids are substituted, deleted, or added in the amino acid sequence of the original helper peptide; and
(b) a peptide consisting of an amino acid sequence in which all or some amino acids, for example, one or more, for example, one, two, three, four, five, six, seven, eight, nine, ten, eleven, twelve, thirteen, fourteen, or fifteen amino acids are modified in the amino acid sequence of the original helper peptide.

The "modification" of amino acids in which the modified helper peptide can have includes, without limitation, for example, acetylation, alkylation such as methylation, glycosylation, hydroxylation, carboxylation, aldehyde formation, phosphorylation, sulfonylation, formylation, modification by aliphatic chain addition such as myristoylation, palmitoylation, or stearoylation, octanolyation, esterification, amidation, deamidation, modification by disulfide bond formation such as cystine modification, glutathione modification, or thioglycolic acid modification, glycosylation, ubiquitination, succinimide formation, glutamylation, and prenylation. The modified helper peptide may include a combination of a substitution, deletion, or addition of at least one amino acid, and a modification of at least one amino acid.

As used herein, the term "cyclooxygenase inhibi tor" means a substance which inhibits a function of a cyclooxygenase (COX). Hereinafter, the cyclooxygenase inhibitor is also called as a "COX inhibitor". The COX inhibitor includes ones which act selectively on a certain cyclooxygenase (for example, COX-1, COX-2), or ones which have no selectivity on it. The COX inhibitor which can be used in the invention includes etodolac, loxoprofen, celecoxib, valdecoxib, parecoxib, lumiracoxib, meloxicam, tenoxicam, diclofenac, mefenamic acid, tolfenamic acid, flufenamic acid, meclofenamic acid, niflumic acid, benzydamine, indobufen, triflusal, tolmetin, fenoprofen, tiaprofenic acid, felbinac, nepafenac, amfenac, pravadoline, zaltoprofen, sulindac, nabumetone, diflunisal, piroxicam, ibuprofen, naproxen, fenoprofen, aspirin, methyl salicylate, salicylamide, salsalate, aloxiprin, tolmetin, indomethacin, proglumetacine, acemetacin, flurbiprofen, pranoprofen, acetaminophen, floctafenine, lornoxicam, tenoxicam, tiaprofenic acid, oxaprozin, ketoprofen, dexketoprofen, dexibuprofen, alminoprofen, ketorolac, mofezolac, phenylbutazone, oxyphenylbutazone, ketophenylbutazone, feprazone, phenbutazone, ethenzamide, tiaramide, tinoridine, epirizole, emorfazone and derivatives thereof, and pharmacologically acceptable salts thereof. In a preferred aspect of the invention, the COX inhibitor is etodolac and/or loxoprofen.

Loxoprofen is represented by the formula:

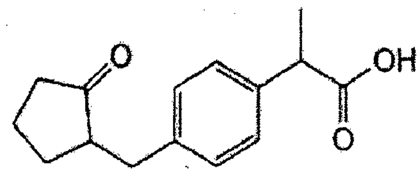

As used herein, the term "prostaglandin receptor antagonist" means a substance which has an inhibitory function on an action of prostaglandin to its receptor, for example, including an EP2 receptor antagonist, an EP4 receptor antagonist, a DP receptor antagonist, and an IP receptor antagonist.

As used herein, the term "EP2 receptor antagonist" means a substance which has an inhibitory function on an action of prostaglandin E2 to an EP2 receptor. The EP2 receptor antagonist includes an AH6809 and derivatives thereof, and pharmacologically acceptable salts thereof.

AH6809 is represented by the formula:

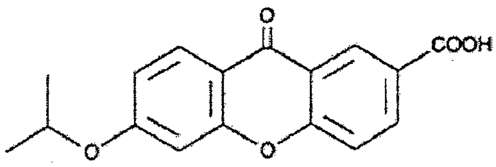

As used herein, the term "EP4 receptor antagonist" means a substance which has an inhibitory function on an action of prostaglandin $E_2$ to an EP4 receptor. The EP4 receptor antagonist includes GW627368X and derivatives thereof, and pharmacologically acceptable salts thereof.

GW627368X is represented by the formula:

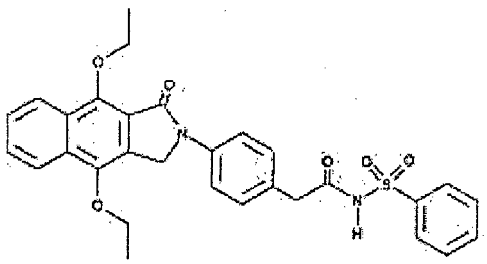

As used herein, the term "DP receptor antagonist" means a substance which has an inhibitory function on an action of prostaglandin $D_2$ to a DP receptor. The DP receptor antagonist includes S-5751, BWA868C and derivatives thereof, and pharmacologically acceptable salts thereof.

BWA868C is represented by the formula:

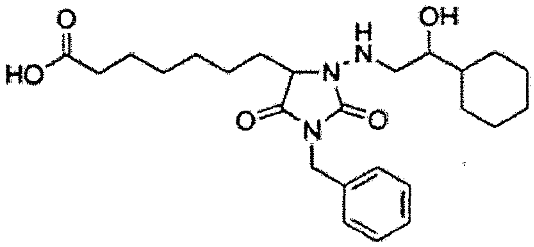

As used herein, the term "IP receptor antagonist" means a substance which has an inhibitory function on an action of prostaglandin $I_2$ to an IP receptor. The IP receptor antagonist includes RO1138452 and derivatives thereof, and pharmacologically acceptable salts thereof.

RO1138452 is represented by the formula:

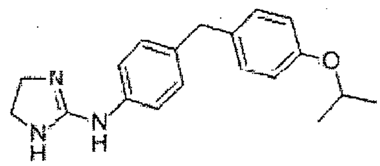

As used herein, the term "prostaglandin receptor agonist" means a substance which has a function of acting on a prostaglandin receptor, for example, including an EP3 receptor agonist.

As used herein, the term "EP3 receptor agonist" means a substance which has a function of acting on an EP3 receptor. The EP3 receptor agonist includes sulprostone, GR63799, cloprostenol, ONO-AE-248, carbacyclin, and derivatives thereof, and pharmacologically acceptable salts thereof.

Sulprostone is represented by the formula:

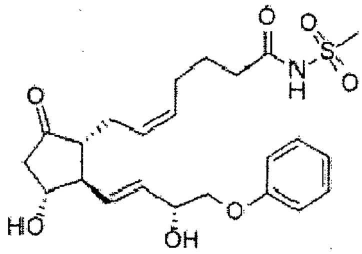

As used herein, the term "TSLP production inhibitor" means a substance which has an inhibitory function on production of TSLP. The TSLP production inhibitor includes naringenin, berberine, resveratrol, luteolin, apigenin, chrysoeriol, vertin, rutin, hesperidin, quercetin, daidzein, genistein, and derivatives thereof, and pharmacologically acceptable salts thereof.

Berberine is represented by the formula:

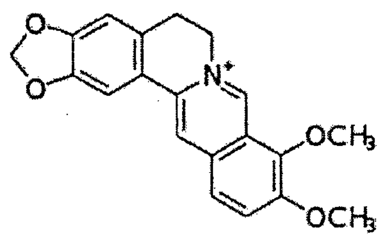

As used herein, the term "adenylate cyclase inhibitor" means a substance which has an inhibitory function on an activity of adenylate cyclase. The adenylate cyclase inhibitor includes 2',5'-dideoxyadenosine, niacin, insulin, and derivatives thereof, and pharmacologically acceptable salts thereof.

2',5'-dideoxyadenosine is represented by the formula:

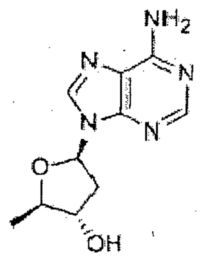

As used herein, the term "omega-3 fatty acid" refers to a member of unsaturated fatty acids having a carbon-carbon double bond at ω-3 position. The omega-3 fatty acid includes eicosapentaenoic acid, α-linolenic acid, docosahexaenoic acid, and derivatives thereof, and pharmacologically acceptable salts thereof.
Eicosapentaenoic acid is represented by the formula:

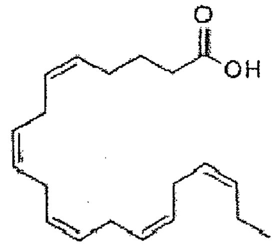

As used herein, the term "PPAR agonist" means a substance which has itselfa function of acting on a peroxisome growth factor-activated receptor, for example, including a PPAR-α agonist, a PPAR-δ agonist, and a PPAR-γ agonist.
As used herein, the term "PPAR-α agonist" means a substance which has a function of acting on an α-type peroxisome growth factor-activated receptor. The term "PPAR-δ agonist" means a substance which has a function of acting on a 5-type peroxisome growth factor-activated receptor. The term "PPAR-γ agonist" means a substance which has a function of acting on a γ-type peroxisome growth factor-activated receptor. The PPAR-α agonist, and/or the PPAR-δ agonist, and/or the PPAR-γ agonist includes clofibrate, fenofibrate, bezafibrate, ciprofibrate, etofibrate, telmisartan, oleylethanolamide, tetradecylthioacetic acid, troglitazone, pioglitazone, rosiglitazone, balaglitazone, rivoglitazone, ciglitazone, darglitazone, edaglitazone, netoglitazone, indeglitazar, tesaglitazar, muraglitazar, aleglitazar, and derivatives thereof, and pharmacologically acceptable salts thereof.
Clofibrate is represented by the formula:

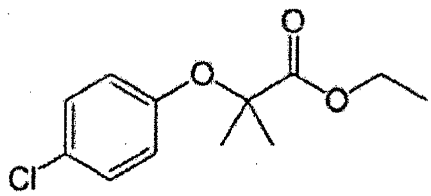

As used herein, the term "dopamine receptor antagonist" means a substance which has an inhibitory function on an action of dopamine to its receptor, for example, including a D1 receptor antagonist, and a D5 receptor antagonist.
As used herein, the term "D1 receptor antagonist" means a substance which has an inhibitory function on an action of dopamine to a D1 receptor. The D1 receptor antagonist includes benzazepine, fenoldopam, lorcaserin, SCH23390, SCH39166, LE300, and derivatives thereof, and pharmacologically acceptable salts thereof.
Benzazepine is represented by the formula:

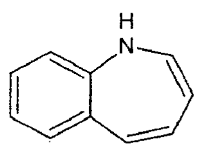

As used herein, the term "D5 receptor antagonist" means a substance which has an inhibitory function on an

action of dopamine to a D5 receptor. The D5 receptor antagonist includes SCH39166 and derivatives thereof, and pharmacologically acceptable salts thereof.

SCH39166 is represented by the formula:

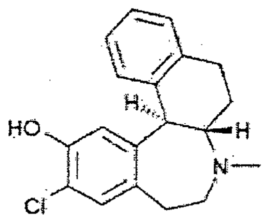

As used herein, the term "dopamine receptor agonist" means a substance which has a function of acting on a dopamine receptor, for example, including a D2 receptor agonist, a D3 receptor agonist, and a D4 receptor agonist.

As used herein, the term "D2 receptor agonist" means a substance which has a function of acting on a D2 receptor. The D2 receptor agonist includes cabergoline, bromocriptine, pergolide, ropinirole, talipexole, aripiprazole, lurasidone, and derivatives thereof, and pharmacologically acceptable salts thereof.

Ropinirole is represented by the formula:

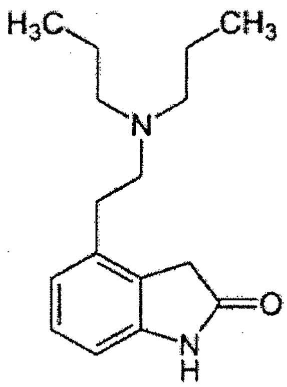

As used herein, the term "D3 receptor agonist" means a substance which has a function of acting on a D3 receptor. The D3 receptor agonist includes piribedil, rotigotine, PD1289077, OH-DPAT, and derivatives thereof, and pharmacologically acceptable salts thereof.

Rotigotine is represented by the formula:

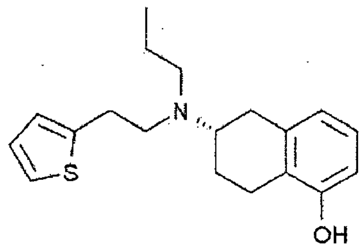

As used herein, the term "D4 receptor agonist" means a substance which has a function of acting on a D4 receptor. D4 receptor agonist includes flibanserin, ABT724, PD168077, CP226269, and derivatives thereof, and pharmacologically acceptable salts thereof.

Flibanserin is represented by the formula:

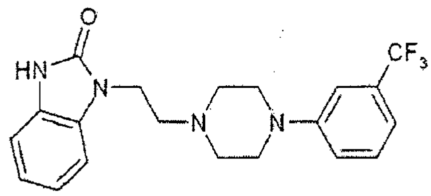

As used herein, the term "histamine receptor antagonist" means a substance which has an inhibitory function on an action of histamine to its receptor, for example, including an H1 receptor antagonist, and an H2 receptor antagonist.

As used herein, the term "H1 receptor antagonist" means a substance which has an inhibitory function on an action of histamine to an H1 receptor. The H1 receptor antagonist includes ketanserin, thonzylamine, mepyramine, tripelenamine, dimethindene, clemastine, bamipine, isothipendyl, chlorphenoxamine, dimetotiazine, chlorpromazine, hydroxyzine, opipramol, betahistine, cinnarizine, levocabastine, antazoline, diphenylpyraline, carbinoxamine, doxylamine, alimemazine, cyclizine, meclozine, levocetirizine, cyproheptadine, phenindamine, triprolidine, azatadine, astemizole, terfenadine, acrivastine, ebastine, desloratadine, rupatadine, bilastine, mizolastine, noberastine, rocastine, temelastine, bepotastine, diphenhydramine, chlorpheniramine, ketotifen, promethazine, cyproheptadine, epinastine, olopatadine, bepotastine, astemizole, emedastine, mequitazine, oxatomide, loratadine, fexofenadine, cetirizine, azelastine, and derivatives thereof, and pharmacologically acceptable salts thereof.

Diphenhydramine is represented by the formula:

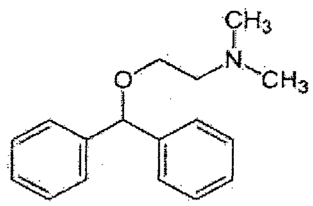

As used herein, the term "H2 receptor antagonist" means a substance which has an inhibitory function on an action of histamine to an H2 receptor. The H2 receptor antagonist includes cimetidine, ranitidine, famotidine, nizatidine, roxatidine, lafutidine, and derivatives thereof, and pharmacologically acceptable salts thereof.

Famotidine is represented by the formula:

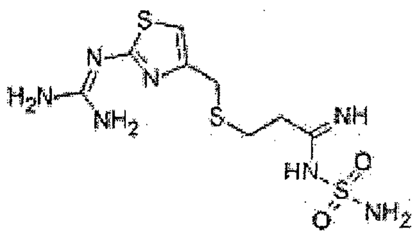

As used herein, the term "histamine receptor agonist" means a substance which has a function of acting on a histamine receptor, for example, an H1 receptor agonist, an H3 receptor agonist, and an H4 receptor agonist.

As used herein, the term "H1 receptor agonist" means a substance which has a function of acting on an H1 receptor. The H1 receptor agonist includes 2-pyridylethylamine, 2-thiazolylethylamine, and derivatives thereof, and pharmacologically acceptable salts thereof.

2-pyridylethylamine is represented by the formula:

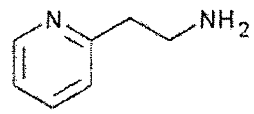

As used herein, the term "H3 receptor agonist" means a substance which has a function of acting on an H3 receptor. The H3 receptor agonist includes Immethridine, Imetit, Immepip, $\alpha$-methylhistamine, proxifan, and derivatives thereof, and pharmacologically acceptable salts thereof.

Proxifan is represented by the formula:

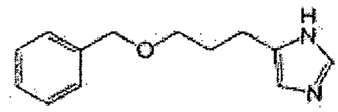

As used herein, the term "H4 receptor agonist" means a substance which has a function of acting on an H4 receptor. The H4 receptor agonist includes 4-methylhistamine, VUF8430, Immepip, and derivatives thereof, and pharmacologically acceptable salts thereof.

4-methylhistamine is represented by the formula:

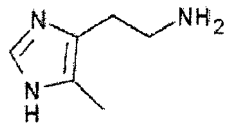

As used herein, the term "serotonin receptor antagonist" means a substance which has an inhibitory function on an action of serotonin to its receptor, for example, including a 5-HT2 receptor antagonist, a 5-HT4 receptor antagonist, a 5-HT6 receptor antagonist, and a 5-HT7 receptor antagonist.

As used herein, the term "5-HT2 receptor antagonist" means a substance which has an inhibitory function on an action of serotonin to a 5-HT2 receptor. The 5-HT2 receptor antagonist includes pizotifen, risperidone, olanzapine, quetiapine, aripiprazole, blonanserin, clozapine, paliperidone, ritanserin, yohimbine, mesulergine, agomelatine, cyclobenzaprine, sarpogrelate, methysergide, ketanserin and derivatives thereof, and pharmacologically acceptable salts thereof.

Olanzapine is represented by the formula:

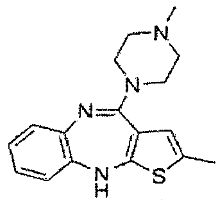

As used herein, the term "5-HT4 receptor antagonist" means a substance which has an inhibitory function on an action of serotonin to a 5-HT4 receptor. The 5-HT4 receptor antagonist includes piboserod, GR113808, GR125487, RS39604, SB204070, and derivatives thereof, and pharmacologically acceptable salts thereof.

Piboserod is represented by the formula:

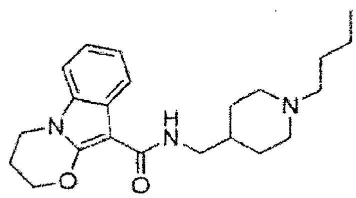

As used herein, the term "5-HT6 receptor antagonist" means a substance which has an inhibitory function on an action of serotonin to a 5-HT6 receptor. The 5-HT6 receptor antagonist includes cerlapirdine, clozapine, and derivatives thereof, and pharmacologically acceptable salts thereof.

Cerlapirdine is represented by the formula:

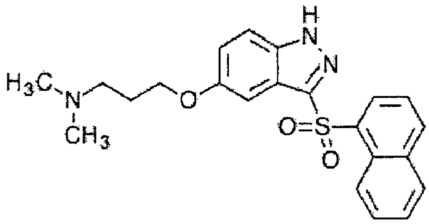

As used herein, the term "5-HT7 receptor antagonist" means a substance which has an inhibitory function on an action of serotonin to a 5-HT7 receptor. The 5-HT7 receptor antagonist includes lurasidone, metergoline and derivatives thereof, and pharmacologically acceptable salts thereof.

Metergoline is represented by the formula:

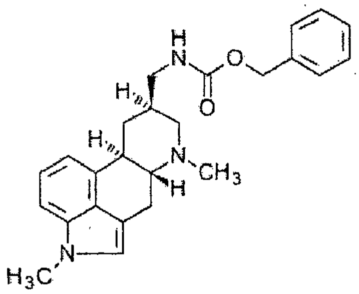

As used herein, the term "serotonin receptor agonist" means a substance which has a function of acting on a serotonin receptor, for example, including a 5-HT1 receptor agonist, and a 5-HT2 receptor agonist.

As used herein, the term "5-HT1 receptor agonist" means a substance which has a function of acting on a 5-HT1 receptor. The 5-HT1 receptor agonist includes piclozotan, tandospirone, sumatriptan, zolmitriptan, eletriptan, rizatriptan, naratriptan, almotriptan, frovatriptan, avitriptan, ergotamine, ergot alkaloid, and derivatives thereof, and pharmacologically acceptable salts thereof.

Zolmitriptan is represented by the formula:

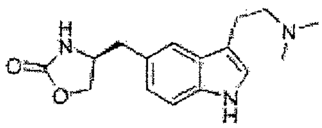

As used herein, the term "5-HT2 receptor agonist" means a substance which has a function of acting on a 5-HT2 receptor. The 5-HT2 receptor agonist includes $\alpha$-methyl-5-HT, agomelatine, norfenfluramine, meta-chlorophenyl piperazine, and derivatives thereof, and pharmacologically acceptable salts thereof.

Agomelatine is represented by the formula:

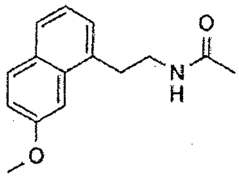

As used herein, the term "vasopressin receptor antagonist" means a substance which has an inhibitory function on an action of vasopressin to its receptor, for example, including a V2 receptor antagonist.

As used herein, the term "V2 receptor antagonist" means a substance which has an inhibitory function on an action of vasopressin to a V2 receptor. The V2 receptor antagonist includes tolvaptan, mozavaptan, conivaptan, lixivaptan, and derivatives thereof, and pharmacologically acceptable salts thereof.

Mozavaptan is represented by the formula:

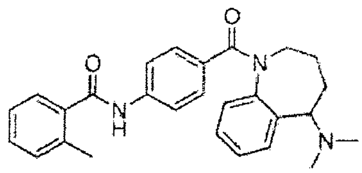

As used herein, the term "vasopressin receptor agonist" means a substance which has a function of acting on a vasopressin receptor, for example, including a V1 receptor agonist.

As used herein, the term "V1 receptor agonist" means a substance which has a function of acting on a V1 receptor. The V1 receptor agonist includes vasopressin, felypressin, desmopressin, lypressin, terlipressin, ornipressin, argipressin, and derivatives thereof, and pharmacologically acceptable salts thereof.

Desmopressin is represented by the formula:

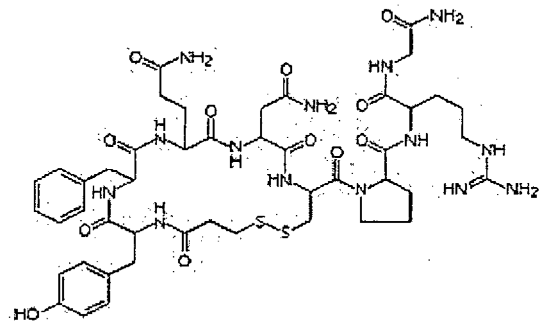

As used herein, the term "muscarine receptor antagonist" means a substance which has an inhibitory function on an action of acetylcholine to a muscarine receptor, for example, an M1 receptor antagonist, an M3 receptor antagonist, and an M5 receptor antagonist.

As used herein, the term "M1 receptor antagonist" means a substance which has an inhibitory function on an action of acetylcholine to a M1 receptor. The term "M3 receptor antagonist" means a substance which has an inhibitory function on an action of acetylcholine to an M3 receptor. The term "M5 receptor antagonist" means a substance which has an inhibitory function on an action of acetylcholine to an M5 receptor. The M1 receptor antagonist, and/or the M3 receptor antagonist, and/or the M5 receptor antagonist includes pirenzepine, atropin, trimebutine, piperidolate, oxybutynin, tropicamide, propiverine, tolterodine, solifenacin, darifenacin, imidafenacin, oxyphencyclimine, tiotropium bromide, S-oxybutynin, tiquizium, and derivatives thereof, and pharmacologically acceptable salts thereof.

Oxybutynin is represented by the formula:

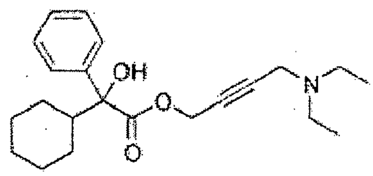

As used herein, the term "muscarine receptor agonist" means a substance which has a function of acting on a muscarine receptor, for example, including an M1 receptor agonist, an M2 receptor agonist, an M3 receptor agonist, an M4 receptor agonist, and an M5 receptor agonist.

As used herein, the term "M1 receptor agonist" means a substance which has a function of acting on an M1 receptor. The term "M2 receptor agonist" means a substance which has a function of acting on an M2 receptor. The term "M3 receptor agonist" means a substance which has a function of acting on an M3 receptor. The term "M4 receptor agonist" means a substance which has a function of acting on an M4 receptor. The term "M5 receptor agonist" means a substance which has a function of acting on an M5 receptor. The M1 receptor agonist, and/or the M2 receptor agonist, and/or the M3 receptor agonist, and/or the M4 receptor agonist, and/or the M5 receptor agonist includes acetylcholine, aceclidine, albamelin, talsaclidine, xanomeline, pilocarpine, cevimeline, bethanechol, mazaticol, muscarine, and derivatives thereof, and pharmacologically acceptable salts thereof.

Bethanechol is represented by the formula:

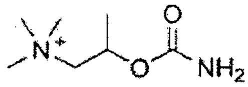

As used herein, the term "adrenergic receptor antagonist" means a substance which has an inhibitory function on an action of adrenaline to its receptor, for example, including an α1 receptor antagonist, a β1 receptor antagonist, a β2 receptor antagonist, and a β3 receptor antagonist.

As used herein, the term "α1 receptor antagonist" means a substance which has an inhibitory function on an action of adrenaline to an α1 receptor. The α1 receptor antagonist includes prazosin, doxazosin, bunazosin, trimazosin, alfuzosin, silodosin, terazosin, tamsulosin, and derivatives thereof, and pharmacologically acceptable salts thereof.

Tamsulosin is represented by the formula:

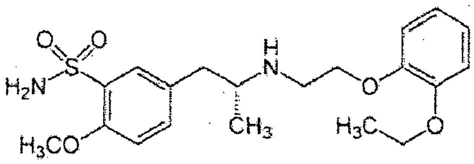

As used herein, the term "β1 receptor antagonist" means a substance which has an inhibitory function on an action of adrenaline to a β1 receptor. The term "β2 receptor antagonist" means a substance which has an inhibitory function on an action of adrenaline to a β2 receptor. The term "β3 receptor antagonist" means a substance which has an inhibitory function on an action of adrenaline to a β3 receptor. The β1 receptor antagonist, and/or the β2 receptor antagonist, and/or the β3 receptor antagonist includes bopindolol, pindolol, timolol, dichloroisoprenaline, alprenolol, carteolol, indenolol, bunitrolol, penbutolol, propranolol, nadolol, nipradilol, tilisolol, acebutolol, celiprolol, metoprolol, atenolol, bisoprolol, betaxolol, practolol, bevantolol, butoxamine, carvedilol, amosulalol, arotinolol, labetalol, and derivatives thereof, and pharmacologically acceptable salts thereof.

Propranolol is represented by the formula:

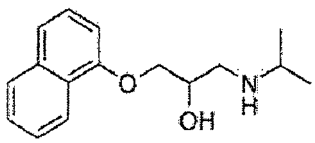

As used herein, the term "angiotensin receptor agonist" means a substance which has a function of acting on an angiotensin receptor, for example, including an AT2 receptor agonist.

As used herein, the term "adrenergic receptor agonist" means a substance which has a function of acting on an adrenergic receptor, for example, including an α1 receptor agonist, and an α2 receptor agonist.

As used herein, the term "α1 receptor agonist" means a substance which has a function of acting on an α1 receptor. The term "α2 receptor agonist" means a substance which has a function of acting on an α2 receptor. The α1 receptor agonist, and/or the α2 receptor agonist includes norepinephrine, norfenefrine, etilefrine, naphazoline, phenylephrine, midodrine, methoxamine, oxedrine, metaraminol, arbutamine, ephedrine, oxymetazoline, tetryzoline, xylometazoline, tramazoline, pseudoephedrine, dipivefrin, aminophylline, methylephedrine, rilmenidine, brimonidine, medetomidine, xylazine, tizanidine, guanfacine, methyldopa, guanabenz, and derivatives thereof, and pharmacologically acceptable salts thereof.

Xylazine is represented by the formula:

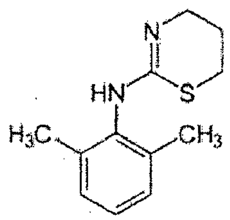

As used herein, the term "angiotensin receptor agonist" means a substance which has a function of acting on an angiotensin receptor, for example, including an AT2 receptor agonist.

As used herein, the term "AT2 receptor agonist" means a substance which has a function of acting on an AT2 receptor. The AT2 receptor agonist includes novokinin, angiotensin, and derivatives thereof, and pharmacologically acceptable salts thereof.

Angiotensin is represented by the formula:

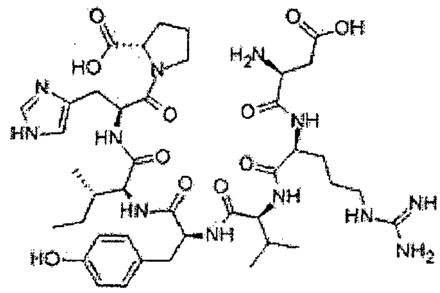

As used herein, the term "GABA receptor agonist" means a substance which has a function of acting on a GABA receptor, for example, including a $GABA_B$ receptor agonist.

As used herein, the term "$GABA_B$ receptor agonist" means a substance which has a function of acting on a $GABA_B$ receptor. The $GABA_B$ receptor agonist includes baclofen, γ-aminobutyric acid, albaclofen, and derivatives thereof, and pharmacologically acceptable salts thereof.

Baclofen is represented by the formula:

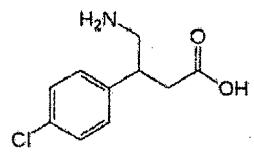

As used herein, the term "thrombin receptor antagonist" means a substance which has an inhibitory function on an action of thrombin to its receptor, for example, including a PAR-1 receptor antagonist.

As used herein, the term "PAR-1 receptor antagonist" means a substance which has an inhibitory function on an action of thrombin to a PAR-1 receptor. The PAR-1 receptor antagonist includes vorapaxar, atopaxar, FR171113, RWJ56110, dabigatran, dabigatran etexilate, melagatran, ximelagatran, hirudin, hirolog, argatroban, and derivatives thereof, and pharmacologically acceptable salts thereof.

Vorapaxar is represented by the formula:

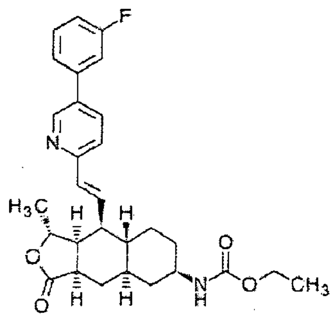

As used herein, the term "thrombin receptor agonist" means a substance which has a function of acting on a thrombin receptor, for example, including a PAR-1 receptor agonist.

As used herein, the term "PAR-1 receptor agonist" means a substance which has a function of acting on a PAR-1 receptor. The PAR-1 receptor agonist includes TRAP-6, TRAP-14, NAT6-$NH_2$ , and derivatives thereof, and pharmacologically acceptable salts thereof.

TRAP-6 is represented by the formula:

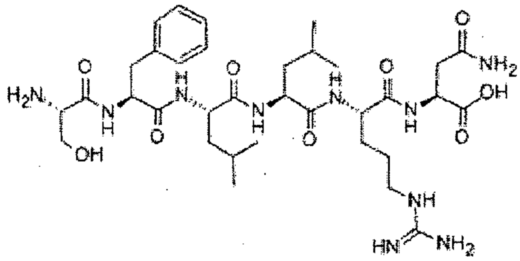

As used herein, the term "opioid receptor agonist" means a substance which has a function of acting on an opioid receptor. The opioid receptor agonist includes trimebutine, alvimopan, morphine, oxycodone, dihydrocodeine, diamorphine, pethidine, pentazocine, buprenorphine, butorphanol, nalbuphine, tilidine, dezocine, meptazinol, tapentadol, naltrexone, methadone, ethylmorphine, hydrocodone, acetyldihydrocodeine, nalorphine, loperamide, remoxipride, opipramol, and derivatives thereof, and pharmacologically acceptable salts thereof.

Buprenorphine is represented by the formula:

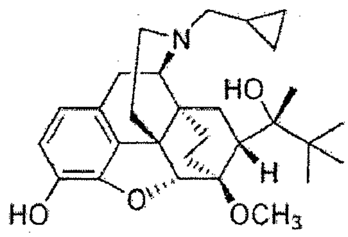

As used herein, the term "leukotriene receptor antagonist" means a substance which has an inhibitory function on an action of leukotriene to its receptor, for example, including a CysLT1 receptor antagonist, and a CysLT2 receptor antagonist.

As used herein, the term "CysLT1 receptor antagonist" means a substance which has an inhibitory function on an action of leukotriene to a CysLT1 receptor. The term "CysLT2 receptor antagonist" means a substance which has an inhibitory function on an action of leukotriene to a CysLT2 receptor. The CysLT1 receptor antagonist, and/or the CysLT2 receptor antagonist includes montelukast, zafirlukast, pranlukast, and derivatives thereof, and pharmacologically acceptable salts thereof. For example, the pharmacologically acceptable salt of montelukast includes montelukast sodium.

Montelukast sodium is represented by the formula:

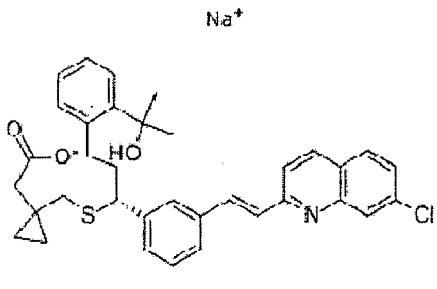

As used herein, the term "leukotriene receptor agonist" means a substance which has a function of acting on a leukotriene receptor, for example, including a BLT receptor agonist.

As used herein, the term "BLT receptor agonist" means a substance which has a function of acting on a BLT receptor. The BLT receptor agonist includes leukotriene B4, CAY10583, and derivatives thereof, and pharmacologically acceptable salts thereof.

Leukotriene B4 is represented by the formula:

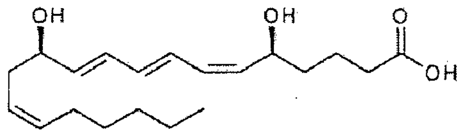

As used herein, the term "ADP receptor agonist" means a substance which has a function of acting on an ADP receptor. The ADP receptor agonist includes adenosine diphosphate, and derivatives thereof, and pharmacologically acceptable salts thereof.

Adenosine diphosphate is represented by the formula:

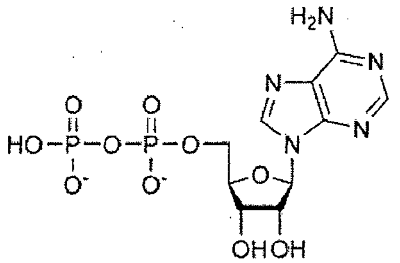

As used herein, the term "melatonin receptor agonist" means a substance which has a function of acting on a melatonin receptor. The melatonin receptor agonist includes melatonin, perlapine, tasimelteon, and derivatives thereof, and pharmacologically acceptable salts thereof.

Melatonin is represented by the formula:

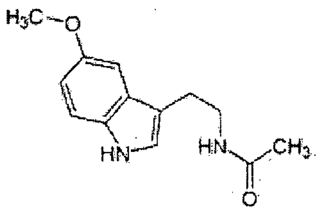

As used herein, the term "somatostatin receptor agonist" means a substance which has a function of acting on a somatostatin receptor. The somatostatin receptor agonist includes somatostatin, somatostatin-14, octreotide, and derivatives thereof, and pharmacologically acceptable salts thereof.

Octreotide is represented by the formula:

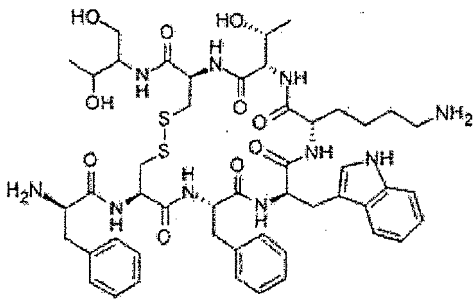

As used herein, the term "cannabinoid receptor agonist" means a substance which has a function of acting on a cannabinoid receptor. The cannabinoid receptor agonist includes dronabinol, nabilone, levonantradol, otenabant, GW833972A, GW405833, and derivatives thereof, and pharmacologically acceptable salts thereof.

Dronabinol is represented by the formula:

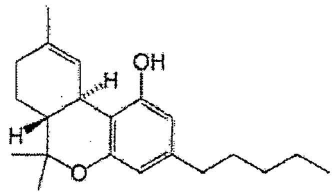

As used herein, the term "sphingosine-1 phosphate receptor agonist" means a substance which has a function of acting on a sphingosine-1 phosphate receptor. The sphingosine-1 phosphate receptor agonist includes fingolimod, and derivatives thereof, and pharmacologically acceptable salts thereof.

Fingolimod is represented by the formula:

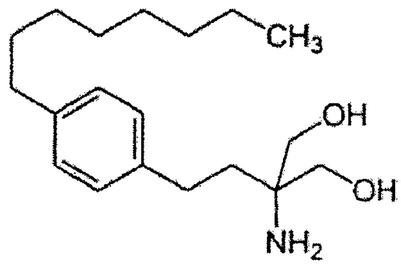

As used herein, the term "metabotropic glutamate receptor agonist" means a substance which has a function of acting on a metabotropic glutamate receptor, for example, including an mGluR2 receptor agonist, an mGluR3 receptor agonist, an mGluR4 receptor agonist, an mGluR6 receptor agonist, an mGluR7 receptor agonist, and an mGluR8 receptor agonist.

As used herein, the term "mGluR2 receptor agonist" means a substance which has a function of acting on an mGluR2 receptor. The term "mGluR3 receptor agonist" means a substance which has a function of acting on an mGluR3 receptor. The term "mGluR4 receptor agonist" means a substance which has a function of acting on an mGluR4 receptor. The term "mGluR6 receptor agonist" means a substance which has a function of acting on an mGluR6 receptor. The term "mGluR7 receptor agonist" means a substance which has a function of acting on an mGluR7 receptor. The term "mGluR8 receptor agonist" means a substance which has a function of acting on an mGluR8 receptor. The mGluR2 receptor agonist, and/or the mGluR3 receptor agonist, and/or the mGluR4 receptor agonist, and/or the mGluR6 receptor agonist, and/or the mGluR7 receptor agonist, and/or the mGluR8 receptor agonist includes VU0361737, VU0155041, biphenylindanone A, PBDA, L-AP4, and derivatives thereof, and pharmacologically acceptable salts thereof.

VU0361737 is represented by the formula:

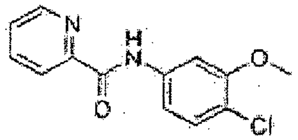

As used herein, the term "phospholipase A2 inhibitor" means a substance which has an inhibitory function on an activity of phospholipase A2. The phospholipase A2 inhibitor includes glycyrrhizic acid, glycyrrhetic acid, and derivatives thereof, and pharmacologically acceptable salts thereof.

Glycyrrhetic acid is represented by the formula:

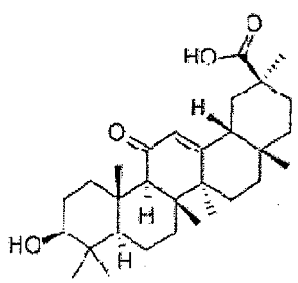

As used herein, the term "TGF-β production inhibitor" means a substance which has an inhibitory function on production of TGF-β. The TGF-β production inhibitor includes pirfenidone, tranilast, and derivatives thereof, and pharmacologically acceptable salts thereof.

Pirfenidone is represented by the formula:

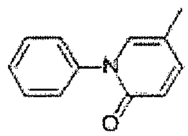

As used herein, the term "Th2 cytokine inhibitor" means a substance which has an inhibitory function on production of a Th2 cytokine such as IL-4 and IL-5. The Th2 cytokine inhibitor includes suplatast and derivatives thereof, and pharmacologically acceptable salts thereof. The pharmacologically acceptable salt of suplatast includes, for example, suplatast tosilate. In a preferred aspect of the invention, the Th2 cytokine inhibitor is suplatast tosilate.

Suplatast tosilate is represented by the formula:

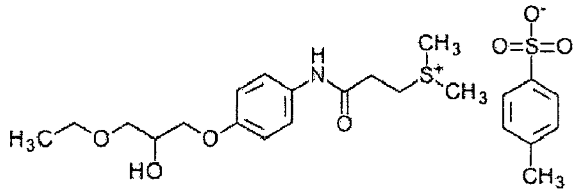

As used herein, the term "immunomodulatory small molecule drug" means a substance which activates or inhibits immune cells, such as T cells, NK cells, or macrophages, except that the substance is not the TLR ligand, the cyclic dinucleotide, the helper peptide, the cyclooxygenase inhibitor, the prostaglandin receptor antagonist, the prostaglandin receptor agonist, the TSLP production inhibitor, the adenylate cyclase inhibitor, the omega-3 fatty acid, the PPAR agonist, the dopamine receptor antagonist, the dopamine receptor agonist, the histamine receptor agonist, the histamine receptor antagonist, the serotonin receptor agonist, the serotonin receptor antagonist, the vasopressin receptor antagonist, the vasopressin receptor agonist, the muscarine receptor antagonist, the muscarine receptor agonist, the adrenergic receptor antagonist, the adrenergic receptor agonist, the angiotensin receptor agonist, the GABA receptor agonist, the thrombin receptor antagonist, the thrombin receptor agonist, the opioid receptor agonist, the ADP receptor agonist, the leukotriene receptor antagonist, the leukotriene receptor agonist, the melatonin receptor agonist, the somatostatin receptor agonist, the cannabinoid receptor agonist, the sphingosine-1 phosphate receptor agonist, the metabotropic glutamate receptor agonist, the phospholipase A2 inhibitor, the TGF-β production inhibitor, or Th2 cytokine inhibitor as described above. The immunomodulatory small molecule drug includes, for example, bestatin, pidotimod, levamisole, golotimod, forphenicinol, and derivatives thereof, and pharmacologically acceptable salts thereof. For example, the pharmacologically acceptable salt of levamisole includes levamisole hydrochloride.

Bestatin is represented by the formula:

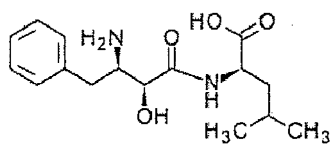

Pidotimod is represented by the formula:

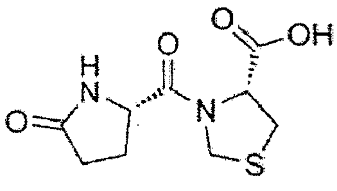

Levamisole hydrochloride is represented by the formula:

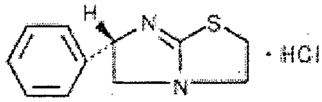

In the invention, the immunomodulatory small molecule drug is generally a compound having a molecular weight of less than 1,000, preferably less than 500. In a preferred aspect of the invention, the immunomodulatory small molecule drug is at least one compound selected from the group consisting of bestatin, pidotimod, and levamisole hydrochloride.

In one aspect, according to the invention, when a desired antigen is administered transdermally or transmucosally, it was found that a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor, and a Th2 cytokine inhibitor are particularly suitably used for inducing cellular immunity. Therefore, in one aspect, the second cellular immunity induction promoter which can be used with the pharmacologically acceptable acid or the pharmacologically acceptable salt thereof in the invention is selected from at least one of them. In a particularly preferred aspect, the second cellular immunity induction promoter is a combination of a helper peptide with at least one selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-β production inhibitor and a Th2 cytokine inhibitor. Various methods for measuring cellular immunity induction quantitatively have been developed, and any one or more methods, for example, ELISPOT method as described in Example may be used.

In a preferred aspect, the second cellular immunity induction promoter contained in the vaccine composition of the invention is at least one selected from the group consisting of a TLR ligand, a cyclic dinucleotide, and a helper peptide, more preferably, a combination of a helper peptide with at least one selected from the group consisting of a TLR ligand and a cyclic dinucleotide. In a particularly preferred aspect, the second cellular immunity induction promoter is a combination of at least one selected from the group of consisting of a TLR4 ligand, a TLR7 and/or a TLR8 ligand, and a cyclic diGMP, and a helper peptide selected from the group of consisting of Peptide-25 and Peptide-25B.

As used herein, non-invasively administration means an administration without applying a physical stimulation, and/or a chemical stimulation, preferably a physical stimulation (for example, tape stripping, microneedle, peeling process, damage process, perforation process) to a skin or a mucous membrane aggressively.

As used herein, the term "mildly irritating condition" means a condition under which irritation to be given to the skin is lower than the irritation generally given in order to improve the skin permeability of the antigen contained in conventional vaccines, or a condition under which irritation is not given to the skin at all. In general, physical and/or chemical stimulation is given to the skin before or simultaneously with the transdermal administration of a conventional vaccine composition so that the antigen can penetrate through the skin. In a preferred aspect of this invention, examples of the mildly irritating condition include a condition with low physical irritation and a condition with low chemical irritation. The condition with low physical irritation is, for example, a condition under which the transepidermal water loss (TEWL) (g/h·m$^2$ in the model animal for evaluating skin irritation is 50 or less, preferably 45 or less, more preferably 40 or less, more preferably 35 or less, still more preferably 30 or less. Because non-treated skin has a TEWL level of about 2 (g/h·m$^2$), the TEWL level before the administration of the vaccine composition may be 2 (g/h·m$^2$) or more. The condition with low chemical irritation is, for example, a condition under which Thymic Stromal Lymphopoietin (TSLP) level (pg/mg protein) in the skin of the model animal for evaluating skin irritation is 10, 000 or less, preferably 9, 000 or less, more preferably 8, 000 or less, more preferably 7,000 or less. Because non-treated skin has a TSLP level of about 1 (pg/mg

protein), the TSLP level at completion of the administration of the vaccine composition is more than 1 (pg/mg protein), preferably more than 2 (pg/mg protein), more preferably more than 3 (pg/mg protein). The "Thymic Stromal Lymphopoietin (TSLP)" is a cytokine involved in the differentiation or recruitment of T cells, the amount can be used as a measurement of the level of the skin stimulation in the invention. Greater TSLP value means stronger skin irritation. Examples of means for accomplishing the condition with low physical irritation include not-conducting the conventional pre-treatment of the skin before the administration such as not-conducting tape stripping or microneedle puncture before the administration. Examples of means for accomplishing the condition with low chemical irritation include avoiding administration of an irritating chemical ingredient such as ethanol or a surfactant at a certain amount or more. The procedure for accomplishing the mildly irritating condition can be determined by using model animals for evaluating skin irritation, and the determined procedure can be applied to the subject to be treated by the vaccine composition, for example, a human subject.

As used herein, the term "cancer" means an abnormal expression of cancer genes, for example, a cancer accompanying an overexpression, for example, a hematopoietic organ tumor or a solid cancer. The cancer gene includes, for example, a survivin gene, a GPC3 gene, an HER2/neu gene, an MAGE3 gene, an MAGE A1 gene, an MAGE A3/A6 gene, an MAGE A4 gene, an MAGE12 gene, a proteinase-3 gene, an AFP gene, a CA-125 gene, a CD44 gene, a CEA gene, a c-Kit gene, a c-met gene, a c-myc gene, an L-myc gene, a COX2 gene, a CyclinD1 gene, a Cytokeratin-7 gene, a Cytokeratin-19 gene, a Cytokeratin-20 gene, an E2F1 gene, an E2F3 gene, an EGFR gene, a Gli1 gene, a hCG$\beta$ gene, an HIF-1$\alpha$ gene, an HnRNP A2/B1 gene, an hTERT gene, an MDM gene, an MDR-1 gene, an MMP-2 gene, an MMP-9 gene, an Muc-1 gene, an Muc-4 gene, an Muc-7 gene, an NSE gene, a ProGRP gene, a PSA gene, a RCAS1 gene, a SCC gene, a thymoglobulin gene, a VEGF-A gene, and a VEGF-A gene. The cancer accompanying the overexpression of the survivin gene includes malignant lymphoma, bladder carcinoma, lung cancer, and colorectal cancer, without limitation. The cancer accompanying the overexpression of the GPC3 gene includes liver cancer, bile duct cancer, and gastric cancer, without limitation. The cancer accompanying the overexpression of the HER2/neu gene includes breast cancer, gastric cancer, ovarian cancer, uterine cancer, bladder carcinoma, non-small cell lung cancer, and prostate cancer, without limitation. The cancer accompanying the overexpression of the MAGE3 gene includes melanoma, lung cancer, head and neck cancer, bladder carcinoma, gastric cancer, esophagus cancer, and liver cancer, without limitation. The cancer accompanying the overexpression of the proteinase-3 gene includes acute myeloid leukemia, and pancreas cancer, without limitation.

As used herein, the term "abnormal expression of a gene" means that the expression level of the gene in a cell is increased or decreased dramatically, for example, by two times or more, for example, 4 times or more as compared with that of other cells in the same tossue. The term "overexpression" means that the abnormal expression is an increase of the expression level. The expression level of the gene can easily be measured by any well-known method in the art.

As used herein, the term "subject" means any animal which can induce an immune response when the vaccine composition is administered in practical use, typically, mammals including human, for example, mouse, rat, dog, cat, rabbit, horse, cow, sheep, pig, goat, monkey, and chimpanzee. The particularly preferred subject is human.

As used herein, the term "model animal for immunological evaluation" means a model animal for evaluating immunity induction properties of the vaccine composition, and specifically means a model animal for evaluating cellular immunity induction level. As the model animal for immunological evaluation used is an animal which can evaluate cellular immunity induction by the antigen contained in the vaccine composition, considering the compatibility of the antigen in the vaccine composition and MHC class 1 molecule of the animal. For example, in the case of the vaccine composition containing an HLA-A* 24 type MHC-restricted class 1 peptide, the evaluation is performed with a BALB/c mouse. In the case of the vaccine composition containing an HLA-A*02 type MHC-restricted peptide, the evaluation is performed wuth a genetically modified mouse which can evaluate cellular immunity induction by the HLA-A* 02 type MHC-restricted peptide. In the case of the vaccine composition containing other HLA type MHC-restricted peptide, the evaluation is performed with an animal which can evaluate cellular immunity induction by the HLA type MHC-restricted peptide. In the case of the vaccine composition containing a protein antigen, the evaluation is performed with an animal having an MHC compatibile with the class 1 epitope to which cellular immunity should be induced among class 1 epitopes contained in the amino acid sequences of the protein antigen. When the fur is cut in order to obtain a site for transdermal administration, an animal after sufficiently curing of the skin damage due to the cutting is used.

As used herein, the term "model animals for evaluating skin irritation" means a model animal for evaluating trans epidermal water loss (TEWL) as a measurement of skin's physical stimulation and TSLP as a skin's stimulation properties of the vaccine composition. Regardless of the type of the antigen contained in the vaccine composition, a C57BL/6 mouse is used as the model animals for evaluating skin irritation. When the fur is cut in order to obtain a site for transdermal administration, an animal after sufficiently curing of the skin damage due to the cutting is used.

As used herein, the term "cancer antigen" means a substance, for example, protein, or peptide, being capable of expressing tumor cells or cancer cells specifically and inducing cellular immune response.

As used herein, the term "cancer antigen peptide" means a partial peptide derived from a cancer antigen protein, and being capable of inducing cellular immunity. Generally, the cancer antigen peptide is generated by decomposing the cancer antigen protein which is a product of the cancer gene in the cancer cell, and the peptide is presented on the

surface of the cancer cell by a MHC class I molecule. The cancer antigen peptide used for the cancer vaccine formulation may be an endogenous cancer antigen peptide isolated or purified from the cancer cell, or a synthetic peptide having the same amino acid sequence as that of the endogenous cancer antigen peptide. In a preferred aspect of the invention, an endogenous cancer antigen peptide or a synthetic cancer antigen peptide, for example, selected from the group consisting of survivin2B peptide and/or a modified survivin-2B peptide, GPC3 peptide and/or a modified GPC3 peptide, HER2/neu_A24 peptide and/or a modified HER2/neu_A24 peptide, MAGE3_A24 peptide and/or a modified MAGE3_A24 peptide, PR1 peptide and/or a modified PR1 peptide, HER2/neu_A02 peptide and/or a modified HER2/neu_A02 peptide, HER2/neu E75 peptide and/or a modified HER2/neu E75 peptide, MAGE3_A02 peptide and/or a modified MAGE3_A02 peptide, and MUC1 peptide and/or a modified MUC1 peptide can be used for cellular immunity induction.

As used herein, the term "virus antigen" means a substance derived from a virus or a component thereof or both of them, the substance being capable of inducing cellular immune response. Therefore, a viral disease can be treated or prevented by administering a virus antigen, preferably with a cellular immunity induction promoter to a subject transdermally. In a preferred aspect of the invention, for example, a peptide selected from the group consisting of IPEP87 peptide and/or a modified IPEP87 peptide and HBVenv peptide and/or a modified HBVenv peptide can be used as a virus antigen.

As used herein, the term "viral disease" means a disease caused by infection, proliferation, and the like of a virus, for example, hepatitis A, hepatitis B, hepatitis C, hepatitis D, hepatitis E, cervical cancer, condyloma acuminatum, HIV infection, Chlamydia infection, and herpes simplex.

II. Vaccine composition for Transdermal Administration

The vaccine composition of the invention for transdermal administration provides high cellular immunity induction in transdermal administration of various antigens by using the antigen with a pharmacologically acceptable acid or a pharmacologically acceptable salt thereof.

As used herein, the term of the pharmaceutical composition "for transdermal administration" means any formation generally used for transdermal administration, and for example, may be a liquid formulation for external use such as a liniment formulation or a lotion formulation, a spray formulation for external use such as aerosol formulation, an ointment, a plaster, a cream formulation, a gel formulation, or an adhesive skin patch, such as a tape preparation, or a cataplasm preparation. The category, definition, properties, production method or the like of the composition is well known in the art, for example, see Japanese Pharmacopoeia Version 16.

For example, a base material for the liniment formulation includes water, ethanol, fatty oils, for example, hard paraffin, soft paraffin, liquid paraffin, glycerin, paraffin oils, beeswax, metal soaps; mucosal fluids (mucilage); natural oils [for example, almond oil, corn oil, peanut oil, castor oil, olive oil or derivatives thereof (for example, polyoxy castor oil)]; mutton suets or derivatives thereof, fatty acids and/or esters thereof (for example, stearic acid, oleic acid, isopropyl myristate).

The lotion formulation is a formation obtained by dispersing active ingredients finely and homogeneously in an aqueous liquid, including a suspended lotion formulation, and an emulsified lotion formulation. Suspending agents include, for example, gum arabic, sodium alginate, sodium carboxymethyl cellulose, methylcellulose, and bentonite. Emulsifying agents include, for example, sodium lauryl sulfate, and sorbitan fatty acid.

For example, as a base material for the ointment, oils and fats, waxes, hydrocarbon compounds or the like as a hydrophobic base can be generally used. Specifically, the base material for the ointment includes mineral bases, such as yellow vaseline, white vaseline, paraffin, liquid paraffin, Plastibase, and silicone, animal or plant bases, such as beeswax and animal fats.

For example, a base material for the cream formulation includes water/oil type base materials, such as hydrophilic ointment, and vanishing cream; oil/water type base materials, such as hydrophilic vaseline, purified lanolin, aquahole, eucerin, neocerin, hydrous lanolin, cold cream, and hydrophilic Plastibase.

For example, as a base material for the gel, carboxy vinyl polymer as a hydrogel base material, gel base, fat-free ointment, polyvinylpyrrolidone, polyvinyl alcohol, sodium polyacrylate, carboxymethyl cellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethyl cellulose, hydroxypropylmethyl cellulose, carboxy vinyl polymer, tragacanth, gum arabic, Tara-Gummi, tamarind seed gum, psyllium seed gum, gelatin, gellan gum, glucomannan, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago seed coat, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl amino acetate, casein, alkyl alginate ester, gelatin, polyethylene glycol can be used.

For example, a base material for the cataplasm preparation includes gelatin, sodium carboxymethyl cellulose,

methylcellulose, sodium polyacrylate, kaolin, polyvinyl alcohol, polyvinylpyrrolidone, glycerin, propylene glycol, and water.

For example, when the tape preparation is a matrix type tape preparation, the tape preparation comprises an adhesive layer having an antigen as an active ingredient, and a support which supports the adhesive layer. When the tape preparation is a reservoir type tape preparation, the tape preparation comprises a reservoir and an adhesive layer having an antigen as an active ingredient, and a support which supports the reservoir and the adhesive layer. The adhesive layer comprises acrylic adhesives, natural rubber adhesives, synthetic rubber adhesives (including rubber elastomer, such as synthetic isoprene rubber, polyisobutylene (PIB), styrene-butadiene rubber, styrene-isoprene-styrene (SIS) rubber), silicone adhesives, vinylester adhesives, vinyl ether adhesives, or the like. The tape preparation comprises an adhesive layer and a support which supports the adhesive layer. If desired, the adhesive layer may be not exposed before use, and the tape preparation may further comprise a release liner which can be easily removed from the adhesive layer when used.

The proportions of the antigen, the pharmacologically acceptable acid or the pharmacologically acceptable salt, and the cellular immunity induction promoter in the pharmaceutical composition of the invention for transdermal administration are not particularly limited. In one aspect, the vaccine composition of the invention for transdermal administration comprises a desired antigen, preferably in an amount of 0.01-40% by weight, more preferably 0.1-30% by weight based on the total weight of the composition. In one aspect, the vaccine composition of the invention for transdermal administration comprises the pharmacologically acceptable acid or the pharmacologically acceptable salt, preferably in an amount of 0.001-30% by weight, more preferably 0.01-20% by weight based on the total weight of the composition. Further, when the vaccine composition of the invention for transdermal administration comprises the cellular immunity induction promoter, the cellular immunity induction promoter is contained preferably in an amount of 0.001-30% by weight, more preferably 0.01-20% by weight, based on the total weight of the composition.

When the vaccine composition of the invention for transdermal administration is the form of a tape preparation, the adhesive layer of the tape preparation (hereinafter also called as "tape preparation of the invention") comprises an antigen and a pharmacologically acceptable acid or a pharmacologically acceptable salt thereof, and further comprises a cellular immunity induction promoter, if desired. In one aspect, the adhesive layer of the tape preparation of the invention comprises the antigen, preferably in an amount of 0.01-40% by weight, more preferably 0.1-30% by weight based on the total weight of the adhesive layer. In one aspect, the adhesive layer of the tape preparation of the invention comprises the pharmacologically acceptable acid or the pharmacologically acceptable salt thereof, preferably in an amount of 0.001-30% by weight, more preferably 0.01-20% by weight based on the total weight of the adhesive layer. When the adhesive layer of the tape preparation of the invention contains the cellular immunity induction promoter, the cellular immunity induction promoter is contained preferably in an amount of 0.001-30% by weight, more preferably 0.01-20% by weight, based on the total weight of the adhesive layer.

The adhesive which should form the adhesive layer of the tape preparation of the invention is not particularly limited, and examples thereof include acrylic adhesives having an acrylic polymer; rubber adhesives having rubber elastomer, such as styrene-diene-styrene block copolymer (for example, styrene-isoprene-styrene block copolymer, styrene-butadiene-styrene block copolymer), polyisoprene, polyisobutylene, butyl rubber, and polybutadiene; silicone adhesives, such as silicone rubber, dimethyl siloxane base, diphenyl siloxane base; vinylether adhesives, such as polyvinylmethylether, polyvinylethylether, and polyvinylisobutylether; vinyl ester adhesives, such as vinyl acetate-ethylene copolymer; and polyester adhesives composed of a carboxylate component, such as dimethyl terephthalate, dimethyl isophthalate, dimethylphthalate and a polyhydric alcohol such as ethylene glycol. The particularly preferred adhesives are acrylic adhesives, rubber adhesives, and silicone adhesives. The adhesive is contained in the adhesive layer preferably in an amount of 10-90% by weight, more preferably 20-80% by weight as the solid basis based on a total amount of the adhesive layer.

An example of the acrylic adhesive is an acrylic ester adhesive comprising as a main component, a polymer of (meth)acrylic acid C2-18 alkyl ester as a first monomer. Examples of the (meth) acrylic acid alkyl ester (first monomer) include a (meth)acrylic acid alkyl ester having a linear, branched, or cyclic alkyl group having a carbon number of 1 to 18 (for example, methyl, ethyl, propyl, butyl, pentyl, hexyl, cyclohexyl, heptyl, octyl, 2-ethylhexyl, nonyl, decyl, undecyl, dodecyl, tridecyl). A (meth)acrylic acid alkyl ester having a linear, branched, or cyclic alkyl group having a carbon number of 4 to 18 is preferred (for example, butyl, pentyl, hexyl, cyclohexyl, heptyl, octyl, 2-ethylhexyl, nonyl, decyl, undecyl, dodecyl, tridecyl). A (meth)acrylic acid alkyl ester having a linear, branched, or cyclic alkyl group having a carbon number of 4 to 8 is more preferred because the use of the monomer component which decreases the glass transition temperature of the polymer is more suitable for providing adhesive properties at ambient temperature (for example, butyl, pentyl, hexyl, cyclohexyl, heptyl, octyl, 2-ethylhexyl, preferably, butyl, 2-ethylhexyl, cyclohexyl, particularly preferably 2-ethylhexyl). Specifically, butyl acrylate, 2-ethylhexyl acrylate, methacrylic acid 2-ethylhexyl, cyclohexyl acrylate, cyclohexyl methacrylate is more preferred. Among them, 2-ethylhexyl acrylate is the most preferred. The alkyl (meth)acrylate ester (first monomer component) can be used alone, or as a combination of two or more.

Further, the acrylic adhesive may contain a second monomer capable of copolymerizing with the alkyl (meth)

acrylate ester as described above. The second monomer includes a monomer having a functional group which can form a linking point when a cross-linker is used. The functional group capable of participating in a crosslink reaction includes a hydroxyl group, a carboxyl group, and a vinyl group. A hydroxyl group and a carboxyl group are preferred. Specific example of the monomer (second monomer component) includes hydroxyethyl (meth)acrylate ester, hydroxypropyl (meth)acrylate ester, N-hydroxyalkyl (meth)acrylamide, (meth) acrylic acid, itaconic acid, maleic acid, maleic acid anhydride, mesaconic acid, citraconic acid, and glutaconic acid. Among them, in view of availability, acrylic acid, methacrylic acid, and hydroxyethyl acrylate ester (in particular, 2-hydroxyethyl acrylate) are preferred, and acrylic acid is the most preferred. The monomer (second monomer component) can be used alone, or as a combination of two or more.

Further, the acrylic adhesive may contain a third monomer in addition to the second monomer, if desired. The third monomer (third monomer component) includes, for example, vinyl esters, such as vinyl acetate, vinyl propionate; vinyl ethers, such as methylvinylether, ethylvinylether; vinylamides, such as N-vinyl-2-pyrolidone, N-vinylcaprolactam; alkoxy (meth)acrylate ester, such as methoxymethyl (meth)acrylate ester, ethoxyethyl (meth)acrylate ester, tetrahydrofurfuryl (meth)acrylate ester; hydroxyl group containing groups, such as hydroxypropyl (meth) acrylate, α-hydroxy methyl acrylate (it is not a linking point as it is used for a third monomer component); (meth)acrylic acid derivatives having an amide group, such as (meth)acrylamide, dimethyl (meth)acrylamide, N-butyl (meth)acrylamide, N-methylol (meth)acrylamide; aminoalkyl ester (meth)acrylate, such as aminoethyl (meth)acrylate ester, dimethylaminoethyl (meth)acrylate ester, t-butylaminoethyl (meth)acrylate ester; alkoxyalkyleneglycol (meth)acrylate ester, such as methoxy ethylene glycol (meth)acrylate ester, methoxy diethylene glycol (meth)acrylate ester, ethoxy polyethylene glycol (meth)acrylate ester, methoxy polypropylene glycol (meth)acrylate ester; (meth)acrylonitrile ; sulfonic acid containing monomers, such as styrene sulfonic acid, allylsulfonic acid, sulfopropyl (meth)acrylate, (meth)acryloyl oxynaphthalene sulfonic acid, acrylamide methylsulfonic acid; vinyl group containing monomers, such as vinylpiperidone, vinylpyrimidine, vinylpiperazine, vinylpyrrol, vinylimidazol, vinyloxazole, vinylmorpholine. Among them, vinyl esters and vinylamides are preferred. The vinyl ester is preferably vinyl acetate, and the vinylamide is preferably N-vinyl-2-pyrolidone. The monomer (third monomer component) can be used alone, or as a combination of two or more.

When the acrylic adhesive is a copolymer comprising an alkyl (meth)acrylate ester (first monomer component) and a vinyl monomer having a functional group capable of participating in a crosslink reaction (second monomer component), the first monomer component and the second monomer component are preferably blended and copolymerized in a ratio by weight of the first monomer component : the second monomer component = 99-85: 1-15, more preferably 99-90 : 1-10.

Further, when the acrylic adhesive is a copolymer comprising an alkyl (meth)acrylate ester (first monomer component), a vinyl monomer having a functional group capable of participating in a crosslink reaction (second monomer component) and another monomer other than the first monomer component and the second monomer component (third monomer component), the first monomer component, the second monomer component, and the third monomer component are preferably blended and copolymerized in a ratio by weight of the first monomer component : the second monomer component : the third monomer component = 40-94 : 1-15 : 5-50, more preferably 50-89 : 1-10 : 10-40.

Polymerization reaction may be performed by any known method, and is not particularly limited. Example includes, for example, a method by adding a polymerization initiator (for example, benzoyl peroxide, azobisisobutyronitrile) to the monomer, and reacting the mixture in a solvent (for example, ethyl acetate) at 50-70°C for 5-48 hours.

The particularly preferred acrylic adhesive in the invention includes, for example, 2-ethylhexyl acrylate ester/acrylic acid/N-vinyl-2-pyrolidone copolymer, 2-ethylhexyl acrylate ester/N-(2-hydroxyethyl)acrylamide/N-vinyl-2-pyrolidone copolymer, a copolymer of 2-ethylhexyl acrylate ester/2-hydroxyethyl acrylate ester/vinyl acetate, 2-ethylhexyl acrylate ester/acrylic acid copolymer, more preferably, 2-ethylhexyl acrylate ester/acrylic acid/N-vinyl-2-pyrolidone copolymer.

If desired, the acrylic adhesive may be physically linked by radiation irradiation including ultraviolet irradiation or electron beam irradiation, or chemically linked with a variety of the cross-linker, for example, isocyanate compounds such as three functional isocyanates or organic peroxides, organic metal salts, metal alcholates, metal chelating compounds, polyfunctional compounds (polyfunctional external cross-linkers, or polyfunctional inner cross-linkers such as diacrylates or dimethacrylates).

The rubber adhesive includes an adhesive including rubber elastomers, for example, polyisobutylene-polybutene elastomer, styrene-diene-styrene block copolymer, styrene-butadiene elastomer, nitrile elastomer, chloroprene elastomer, vinylpyridine elastomer, polyisobutylene elastomer, butyl elastomer, isoprene-isobutylene elastomer. Among them, polyisobutylene (PIB), styrene-diene-styrene block copolymer [for example, styrene-butadiene-styrene block copolymer (SBS), styrene-isoprene-styrene block copolymer (SIS)] are preferably used in view of solubility in peptides and the cellular immunity induction promoters and tissue adhesion. They may be used in mixture.

Further, the rubber adhesive can be used by mixing two or more rubber elastomers having different average molecular weights in order to obtain a suitable adhesiveness and solubility of agents, and the rubber elastomers may be same component or different components. Considering a polyisobutylene as an example, a mixture of a high molecular weight polyisobutylene having an average molecular weight of 150,000-5,500,000 and a medium molecular weight

polyisobutylene having an average molecular weight of 10,000-150,000 and/or a low molecular weight polyisobutylene having an average molecular weight of 500-4,000 is preferred. In this case, the high molecular weight polyisobutylene, the medium molecular weight polyisobutylene, and the low molecular weight polyisobutylene are suitably blended in a weight ratio of high molecular weight : medium molecular weight : low molecular weight=10-80, preferably 20-70 : 0-90, preferably 10-80 : 0-80, preferably 10-60.

The average molecular weight in the invention means a viscosity average molecular weight calculated from Flory viscosity equation. The average molecular weight is calculated by calculating Staudinger index ($J_0$) by Schulz-Blaschke equation from a flow time resulted from a capillary 1 on an Ubbelohde type viscometer at 20°C, and then using the $J_0$ value in the following formula.

(Formula)

$$J_0 = \eta_{sp}/c(1 + 0.31\eta_{sp}) \text{ (Schulz-Blaschke equation)}$$

$$\eta_{sp} = t/t_0 - 1$$

t: Flow time of solution (according to Hagenbach-couette correction formula)
to: Flow time of solvent (according to Hagenbach-couette correction formula)
c: Concentration of solution (g/cm$^3$)

$$J_0 = 3.06 \times 10^{-2} \overline{Mv}^{0.65}$$

$\overline{Mv}$: Viscosity average molecular weight

The rubber adhesive may contain tackifiers, for example, rosin resins, polyterpene resins, coumarone-indene resins, petroleum based resins, terpene-phenol resins, xylene resins, and alicyclic saturated hydrocarbon resins in order to obtain a suitable adhesiveness. One, two or more tackifiers can be blended in a proportion of 50% by weight or less, preferably 5-40% by weight based on a total weight of the rubber adhesive.

The silicone adhesive includes silicone adhesives selected from polyorganosiloxane adhesives, polydimethylsiloxane adhesives, or polydimethyl diphenyl-siloxane adhesives. Among them, any commercially available silicone adhesive, for example, BIO PSA from Dow Corning Corporation is preferably used.

Although the support which supports the adhesive layer is not particularly limited, preferred are a substance having an imperviableness to a peptide or a cellular immunity induction promoter virtually, that is, a support which prevents a peptide, a cellular immunity induction promoter, an additive and the like contained in the adhesive layer from passing through the support to release them from the back side of the support and decrease their contents.

As the support, for example, a single film of polyester, polyamide, polychlorovinylidene, polyethylene, polypropylene, polyvinylchloride, ethylene-ethyl acrylate copolymer, polytetrafluoroethylene, ionomer resin, metal foil, or their laminated film can be used. Among them, preferred is a support of a laminated film comprising a non-porous plastics film made of the above-mentioned materials and a porous film in order to improve the adhesiveness (anchoring property) between the support and the adhesive layer. In this case, the adhesive layer is preferably formed at the side of the porous film. As the porous film, the film which improves the anchoring property to the adhesive layer is selected. Specifically, the film includes paper, woven fabric, non-woven fabric, knitted fabric, and a sheet treated mechanically by the perforation process. Among them, particularly preferred are paper, woven fabric, and non-woven fabric in view of handling property. The porous film having a thickness of 1-200 μm is selected in view of improvement of anchoring property, flexibility of the tape preparation and handling property of application. Further, when woven fabric or non-woven fabric is used as the porous film, the weight per unit area is preferably 5-30 g/m$^2$, more preferably 6-15 g/m$^2$.

The most suitable support is a laminated film comprising a polyester film having a thickness of 1.5-6 μm (preferably, polyethylene phthalate film) and a non-woven fabric made of a polyester (preferably, polyethylene terephthalate) having a weight per unit area of 6-15 g/m$^2$.

In the tape preparation of the invention, a release liner is preferably laminated to the adhesive face of the adhesive layer in order to protect the adhesive face before use. The release liner is not particularly limited, as long as the liner has a sufficiently low release force by releasing process. For example, used are films such as polyester, polyvinylchloride, polyvinylidene chloride, polyethylene terephthalate; papers such as high quality paper or glassine

paper; or laminated films comprising a quality paper or glassine paper and polyolefin, which are treated by releasing process by applying silicone resins or fluorine resins on the contact surface to the adhesive layer. The release liner preferably has a thickness of 10-200 μm, more preferably 25-100 μm. The release liner is preferably made of polyester (in particular, polyethylene terephthalate) resin in view of barrier property, cost, and the like. Further, in this case, the liner preferably has a thickness 25-100 μm in view of handling property.

III. Vaccine Composition for Transmucosal Administration

The vaccine composition of the invention for transdermal administration provides high cellular immunity induction in transdermal administration of various antigens by using the antigen together with a pharmacologically acceptable acid or a pharmacologically acceptable salt thereof.

As used herein, the term of the composition "for transmucosal administration" may be, for example, any formulations generally used for transmucosal administration, for example, sublingual, nasal, buccal, rectal or vaginal administration, for example, half-solid formulations, such as a gel formulation (a jerry formulation), a cream formulation, an ointment, a plaster, liquid formulation, solid formulations, such as powder, fine granules, granules, film formulation or tablet, orally-disintegrating tablet, spray formulations for mucous membrane, such as aerosol formulation, an aspirator. The category, definition, properties, production method or the like of the composition is well known in the art, for example, see Japanese Pharmacopoeia Version 16.

For example, as a solvent for liquid formulation, solvent such as a suitable amount of water or ethanol, glycerin, propylene glycol or the like can be used. The components of the composition of the invention can be dispersed or dissolved in the solvent to prepare the liquid formulation.

For example, as a base material for the gel formulation (the jerry formulation), carboxy vinyl polymer as a hydrogel base material, gel base, fat-free ointment, polyvinylpyrrolidone, polyvinyl alcohol, sodium polyacrylate, carboxymethyl cellulose, starch, xanthan gum, karaya gum, sodium alginate, methylcellulose, hydroxypropylcellulose, hydroxypropylmethylcellulose phthalate (HPMCP), cellulose acetate phthalate (CAP), carboxymethylethylcellulose (CMEC), ethylcellulose, hydroxyethyl cellulose, hydroxypropylmethyl cellulose, carboxy vinyl polymer, tragacanth, gum arabic, Tara-Gummi, tamarind seed gum, psyllium seed gum, gelatin, gellan gum, glucomannan, locust bean gum, guar gum, carrageenan, dextrin, dextran, amylose, potassium carboxymethyl cellulose, sodium carboxymethyl cellulose, calcium carboxymethyl cellulose, pullulan, chitosan, sodium carboxymethyl starch, Plantago seed coat, galactomannan, Eudragit, casein, alkyl alginate ester, gelatin, polyethylene glycol can be used. The base material can be dissolved in a solvent to prepare a gel formulation having a flowability or formability. As a solvent, water is preferred, but glycerin or propylene glycol can be also used.

For example, a base material for the cream formulation includes water/oil type base materials, such as hydrophilic ointment, and vanishing cream; oil/water type base materials, such as hydrophilic vaseline, purified lanolin, aquahole, eucerin, neocerin, hydrous lanolin, cold cream, and hydrophilic Plastibase. The base material can be agitated in an oleaginous solvent or water with a homogenizer at high speed to prepare a cream formulation.

For example, a base material for the film formulation includes polyvinylpyrrolidone, polyvinyl alcohol, sodium polyacrylate, carboxymethyl cellulose, starch, xanthan gum, karaya gum, sodium alginate, methylcellulose, carboxy vinyl polymer, agar, hydroxypropylcellulose, hydroxypropylmethylcellulose phthalate (HPMCP), cellulose acetate phthalate (CAP), carboxymethylethylcellulose (CMEC), ethylcellulose, hydroxyethylcellulose, hydroxypropylmethylcellulose, carboxy vinyl polymer, tragacanth, gum arabic, locust bean gum, guar gum, carrageenan, dextrin, dextran, amylose, carboxymethyl cellulose potassium, sodium carboxymethyl cellulose, calcium carboxymethyl cellulose, pullulan, chitosan, sodium carboxymethyl starch, Plantago seed coat, galactomannan, aminoalkyl methacrylate copolymer E, aminoalkyl methacrylate copolymer RS, methacrylic acid copolymer L, methacrylic acid copolymer LD, methacrylic acid copolymer S, methyl acrylate-methacrylic acid-methyl methacrylate copolymer, ethyl acrylate-methyl methacrylate copolymer, polyvinyl acetal diethyl amino acetate, casein, and alkyl alginate ester. The base material can be dissolved in a polar organic solvent such as water or ethanol, and then subjected to film coating formation and dried to prepare a film formulation. In one preferred aspect, the vaccine composition of the invention for transmucosal administration is a film formulation form.

For example, powder, fine granules, granules, film formulation or tablet can be prepared by using excipients such as lactose, cornstarch, microcrystalline cellulose; binders such as hydroxypropylcellulose, gum Arabic as an additive, and further using a suitable amount of solvents such as water or ethanol, stirring the mixture, and then shaping the mixture by granulation, drying, or compression. If needed, lubricants such as magnesium stearate, or coating agents such as hydroxypropylcellulose, sucrose may be added to the mixture.

For example, a base material for the orally-disintegrating tablet (freeze dry type) includes polysaccharides such as gelatin or pullulan. As a forming aid, mannitol, trehalose, sorbitol, glycine, or the like may be used. The orally-disintegrating tablet (freeze dry type) can be prepared by dissolving the additive in water to form a solution, dispensing the solution, and freeze drying the solution. In one preferred aspect, the vaccine composition of the invention for transmucosal administration is an orally-disintegrating

tablet form.

For example, the aerosol formulation includes a liquid formulation, a gel agent having high flowability, a cream formulation, and fine powders such as a powder as a content. The aerosol formulation can be administered efficiently to a site of administration, for example, oral mucosa or nasal mucosa by dispersing the solid or liquid fine particles of the content into gas with an atomizing device.

The proportions of the antigen, the pharmacologically acceptable acid or the pharmacologically acceptable salt thereof, and the cellular immunity induction promoter in the vaccine composition of the invention for transmucosal administration are not particularly limited. In one aspect, the vaccine composition of the invention for transmucosal administration comprises a desired antigen, preferably in an amount of 0.01-40% by weight, more preferably 0.1-30% by weight based on the total weight of the composition. In one aspect, the vaccine composition of the invention for transmucosal administration comprises the pharmacologically acceptable acid or the pharmacologically acceptable salt thereof, preferably in an amount of 0.001-30% by weight, more preferably 0.01-20% by weight based on the total weight of the composition. Further, when the vaccine composition of the invention for transmucosal administration contains the cellular immunity induction promoter, the cellular immunity induction promoter is contained preferably in an amount of 0.001-30% by weight, more preferably 0.01-20% by weight based on the total weight of the composition.

Further, the composition of the invention for transdermal or transmucosal administration may optionally contain any additives. The additive may be, for example, an isotonizing agent, preservative and bactericidal agent, antioxidant, solubilizer, solubilization aid, suspending agent, filler, pH modifying agent, stabilizing agent, absorption promoter, sustained release preparation, coloring agent, plasticizer, cross-linker, adhesive, or a combination of these two or more additives depending on the compatibility with the major component of a base material, an antigen, a pharmacologically acceptable acid or a pharmacologically acceptable salt thereof and a cellular immunity induction promoter, and administration regimen to be intended. Further, when the composition of the invention is a tape preparation, the tape preparation can comprise a skin permeability enhancer as an additive.

As used herein, the term "skin permeability enhancer" means any substance which improves the permeation efficiency of an antigen transdermaly administrated with the substance into a skin as compared with administration of the same antigen without the substance. The skin permeability enhancer is not particularly limited, as long as the enhancer is liquid or flowable at room temperature (25°C), or when a mixture of two or more enhancers are used, the mixture is a liquid at room temperature (25°C) and have a proabsorptive effect. The organic liquid component is preferably hydrophobic liquid component in view of the compatibility of the adhesive layer. The skin permeability enhancer includes for example, higher alcohols, such as oleyl alcohol, octyldodecanol; polyhydric alcohol, such as glycerin, ethylene glycol, polypropylene glycol; higher fatty acids, such as oleic acid, caprylic acid; fatty acid esters, such as isopropyl myristate, isopropyl palminate, ethyl oleate; polybasic acid esters, such as diethyl sebacate, diisopropyl adipate; polyhydric alcohol fatty acid esters, such as triisostearic acid diglyceryl, monooleic acid sorbitan, dicaprylic acid propylene glycol, monolauric acid polyethylene glycol, tetraoleic acid polyoxyethylene sorbit; polyoxyethylenealkylethers, such as polyoxyethylenelaurylether; hydrocarbons, such as squalane, liquid paraffin; plant oils, such as olive oil, castor oil; silicone oil; pyrolidones, such as N-methylpyrolidone, N-dodecylpyrolidone; sulfoxides, such as decylmethylsulfoxide. They can be used alone, or as a combination of two or more.

When rubber adhesives or acrylic adhesives are used, a second skin permeability enhancer can be used. Specific example of the second skin permeability enhancer includes polyvinylpyrrolidone, crospovidone, polypropylene glycol, polyvinyl alcohol, carboxy vinyl polymer, hydroxypropylcellulose, or mixture thereof, without limitation. In a preferred aspect, the second skin permeability enhancer of the invention is polyvinylpyrrolidone, crospovidone and/or polypropylene glycol.

In view of improving skin permeability of the antigen peptide, the skin permeability enhancer which is preferably used includes higher alcohols, more specifically, higher alcohols having a carbon number of 8-18 (preferably 8-14), fatty acid esters, more specifically, fatty acid esters of a fatty acid having a carbon number of 8-18 (preferably 12-16) and a monovalent alcohol having a carbon number of 1-18, polyhydric alcohol fatty acid esters, in particular, fatty acid esters, in particular, isopropyl myristate, isopropyl palmitate, and diethyl sebacate. The amount of the skin permeability enhancer is preferably from 0.1% by weight to 70% by weight, more preferably from 1% by weight to 65% by weight, more preferably from 5% by weight to 60% by weight based on a total amount of the adhesive layer. When the proportion of the skin permeability enhancer is 0.1% by weight or more, high transdermal proabsorptive effect is obtained. When the proportion is 70% by weight or less, high transdermal proabsorptive effect is advantageously obtained while preventing the decrease of total adhesiveness and cohesion force of the adhesive layer.

The composition of the invention for transdermal administration is preferably administered to a subject under mildly irritating condition. The administration under mildly irritating condition can be accomplished by, for example, (i) administering the composition of the invention for transdermal administration to a subject in a condition in which transepidermal water loss (TEWL) (g/h·m$^2$) is 50 or less as evaluated by model animals for evaluating skin irritation, (ii) administering the composition of the invention for transdermal administration to a subject in a condition in which cutaneous TSLP level (pg/mg protein) is 10,000 or less as evaluated by model animals for evaluating skin irritation.

The therapeutically effective amount of the antigen can vary widely depending on a severity of the disease, an age or relative health of the subject, and other known factors. The therapeutically effective amount is from about 0.1 μg to about 1 g per kg of the body weight of the subject per day for satisfactory result. The pharmacologically acceptable acid or the pharmacologically acceptable salt thereof can be administered with the antigen at the same time or sequentially, preferably at the same time. The effective amount of the pharmacologically acceptable acid or the pharmacologically acceptable salt thereof can vary widely depending on a particular acid or salt used, the presence of another cellular immunity induction promoter used together, and the like. The effective amount is from about 0.01 μg to about 1 g per kg of the body weight of the subject per day for satisfactory result. When the cellular immunity induction promoter is used together, the cellular immunity induction promoter can be administered with the antigen at the same time or sequentially, preferably at the same time. The effective amount of the cellular immunity induction promoter can vary widely depending on a particular cellular immunity induction promoter used, the presence of another cellular immunity induction promoter, and the like. The effective amount is from about 0.01 μg to about 1 g per kg of the body weight of the subject per day for satisfactory result. The daily dosage may be administered as a single dose or two or more doses, for example, in divided doses of two, three, four, or five times daily. The continuous dosing period is suitably selected from 1 minute to 7 days. The dose interval is suitably selected from daily to yearly (for example, daily, one per two days, one per three days, weekly, one per two weeks, monthly, one per three months, one per six months, yearly) or longer interval, depending on a condition of the patient, a severity of the disease, for treatment or prevention, and the like. Generally, for treating the patient having a severe disease, an antigen is administered with higher frequency in higher doses. For preventing the patient without the disease, an antigen is administered with lower frequency in lower doses.

The invention will be described in more detail and more specifically in the following Examples, but is not limited to the scope of the Examples.

## EXAMPLES

### Tape Preparation for Transdermal Administration

The adhesive used for tape preparations were prepared.

(Acrylic Adhesive A)

An acrylic adhesive A solution was obtained by solution polymerization of 75 parts of 2-ethylhexyl acrylate, 22 parts of N-vinyl-2-pyrrolidone, 3 parts of acrylic acid and 0.2 part of azobisisobutyronitrile in ethyl acetate in inert gas atmosphere at 60°C.

(PIB rubber adhesive)

A PIB rubber adhesive solution was obtained by dissolving 24 parts of a polyisobutylene (Oppanol B200, manufactured by BASF), 36 parts of a polyisobutylene (Oppanol B12, manufactured by BASF), and 40 parts of an alicyclic petroleun polymer resin (ARKON P-100, manufactured by ARAKAWA CHEMICAL INDUSTRIES, LTD.) in toluene.

The tape preparations having compositions as shown in Tables 1-3 were prepared. Specifically, the adhesive solution, an antigen peptide, a pharmacologically acceptable acid, a cellular immunity induction promoter, a skin permeability enhancer, an organic solvent (for example, ethyl acetate), and optionally an additional additive were mixed and blended so that the concentration of each component after drying organic solvents was in an amount as shown in Tables 1-3 to form a mixture, and spreaded on a release liner so that the thickness of the mixture after drying organic solvents was about 80 μm. The mixture was then dried to remove any organic solvent, and attached to a support to form a tape preparation. The adhesive solution was blended so that total amount of each components and the adhesive is 100 parts by weight after drying. The tape preparation was cut into a piece having an area of 0.7 cm$^2$. The piece was used as a sample for immunity induction study. When administered, the release liner was removed off and the tape preparation was administered. A polyethylene terephthalate (PET) film (thickness 25 μm) was used as the support. A liner made of polyethylene terephthalate (PET) (thickness 75 μm) after silicone releasing process was used as the release liner. Imiquimod (IMQ) was purchased from Tokyo Chemical Industry Co., Ltd. HER2/neu E75 peptide (TFAsalt), IPEP87 peptide (TFA salt), PR1 peptide (TFA salt), Peptide-25 (TFA salt) and PADRE (acetate) were all prepared by chemical synthesis, and purified by HPLC before use.

Immunity Induction Study 1 in Mouse (Tape Preparation)

The tape preparations for external use as prepared in the above were used to examine a mouse immunity induction study using model animals for immunological evaluation. The immunity induction level was evaluated by ELISPOT method. Specifically, the fur of the back skin of a mouse was cut, and the mouse was kept in a rearing period for curing of the skin damage. After the period, a sample was administered to the back skin of the mouse for a predetermined period, and then removed. After a predetermined day, the level of the cellular immunity induction specific for the antigen was evaluated. On a predetermined day after the administration, the spleen of the mouse was removed to prepare a suspension of the spleen cells. Spleen cells ($3\times10^6$ cells/well) and an antigen (100 μM for peptide antigen, 100 μg/mL for protein antigen) were added with a culture solution in a well of ELISPOT plate containing a fixed anti-mouse IFN-γ antigen, and co-cultured at 37°C in 5% $CO_2$ for 20 hours. The number of spots of IFN-γproducing cells was evaluated by ELISPOT method (the number of the spot/$3\times10^6$ cells). The number of the administration was one (24hr/week), the removal of the spleen was 6 days after the administration.

For some tape preparation, according to the method as described below, using a model animal for skin irritation evaluation, cutaneous TSLP level in the skin of the animal (mouse) after administration and transepidermal water loss in the animal (mouse) before administration were measured.

(Method for Measuring TSLP Level)

At the completion of the formulation administration, the back skin of the mouse was removed, and the skin was disrupted in an extraction solvent (a PBS solution containing a protease inhibitor (Protease Inhibitor Cocktail for general use, SIGMA-ALDRICH) and 10 μM indomethacin (Wako Pure Chemical Industries, Ltd.)) using a homogenizer (Physcotron, MICROTEC CO., LTD.). The disrupted skin was centrifuged at 9,000 X g at 4°C for 10 minutes, and then the supernatant was collected. The amount of TSLP in the supernatant was measured by ELISA (Mouse TSLP Quantikine ELISA Kit, R&D Systems). Further, the amount of total proteins in the supernatant was measured by BCA method (Pierce BCA protein Assay Kit, Thermo SCIENTIFIC). The amount of TSLP was divided by the amount of total proteins for normalization.

(Measurement of Transepidermal Water Loss)

A portable machine for measuring the water loss in closed chamber method (manufactured by AsahiBioMed Co., Ltd., VAPO SCAN AS-VT100RS) was used. The machine was contacted with the skin of a mouse for 5-15 seconds for measurement. Ten minutes after the pretreatment of the skin of the mouse, the water loss was measured, and the obtained value was considered as a transepidermal water loss (TEWL) ($g/h{\cdot}m^2$).

The results of the immunity induction study, and the measurement results of TSLP level and transepidermal water loss, as well as the mouse used are shown in Tables 1-3. In the Tables, "genetically modified mouse" is a genetically modified mouse in which the cellular immunity induction by HLA-A*0201 type MHC-restricted peptide can be evaluated. In addition, for the comparison, the result of the immunity study using injections as described later (Comparative Example 2) is shown at the bottom of the Tables.

<u>In vivo CTL assay</u>

Seven days after final immunity administration, a spleen cell (target cell or control cell) was transplanted according to the following procedure, a spleen was collected after 18 hours, and % Specific Lysis was obtained by performing FACS measurement.

Procedure 1. Collection of spleen cell of naïve mouse

A spleen was isolated from a naive mouse (C57BL/6), and was mashed using a glass slide in a petri dish containing a RPMI1640 medium. After recovery into a 50 mL tube, the mashed sample was centrifuged at 10°C and 1100 rpm for 5 minutes, the supernatant was discarded, and 20 mL of a Lysis Buffer was added, followed by incubation at room temperature for 5 minutes. After 20 mL of a medium was added, the resultant was centrifuged, a medium was added, and the resultant was passed through a cell strainer.

Procedure 2. Labeling of spleen cell with antigen

After the spleen cell prepared by Procedure 1 was centrifuged at 10°C and 1100 rpm for 5 minutes, the supernatant was discarded, and a HBSS buffer was added to $2\times10^7$ cells/mL. This cell solution was dispensed into two 50 mL tubes, 100 μM of an antigen solution (the antigen was an antigen which was blended into each immunity administration product) was added to one of the tubes containing the cell solution so that the final concentration became 10 μM, to

obtain a target cell. The cell in another tube was adopted as a control cell. The cells in both tubes were incubated at 37°C for 1 hour, centrifuged, the supernatant was discarded, and a medium was added.

Procedure 3. Labeling of spleen cell with CFSE

The cell labelled with an antigen according to Procedure 2 was centrifuged, and 0. 1% BSA-PBS was added to $1 \times 10^7$ cells/mL. To the target cell solution was added a 5 mM CFSE solution to a final concentration of 10 μM, and to the control cell solution was added a 5 mM CFSE solution to a final concentration of 1 μM, and the mixture was vortexed, followed by incubation at 37°C for 10 minutes. Thereafter, centrifugation was performed, the supernatant was discarded, and a medium was added.

Procedure 4. Transplantation of spleen cell

The cell labelled with CFSE according to Procedure 3 was centrifuged, the supernatant was discarded, and cells were prepared to $5 \times 10^7$ cells/mL using a HBSS buffer. Equal amounts of the target cell solution and the control cell solution were mixed, and each 200 μL was administered to an immunized mouse via orbital veins (transplantation cell number: $1 \times 10^7$ cells/animal).

Procedure 5. Preparation of spleen cell of immunized mouse and measurement of FACS

Eighteen hours after transplantation of the spleen cell, a spleen was isolated, and a spleen cell was prepared in the same manner as in Procedure 1. Thereafter, a CFSE-positive cell was detected by FACS, and the cytotoxic activity was evaluated by the following expression from the ratio between a CFSE high cell (target cell) and a CFSE low cell (control cell). By this index, the ability of antigen-specifically induced immunity to specifically attack an antigen presenting cell in a living body was evaluated, and it was confirmed that an administration product of the present invention has the high effect.

$$r = (\%\ \text{CFSE low cells})/(\%\ \text{CFSE high cells})$$

$$\%\ \text{Specific Lysis} = (1 - (r_{_\text{non immunized}}/r_{_\text{immunized}})) \times 100$$

Table 1

| | Composition | | | | | | | TSLP (pg/mg protein) | physical irritation | TEWL (g/h·m$^2$) | mouse for immmological evaluation | Results of immunization (ELISPOT average spot number) | % Specific Lysis (In vivo CTL assay) |
|---|---|---|---|---|---|---|---|---|---|---|---|---|---|
| | base | antigen peptide | cellular immunity induction promoter | | skin permeability enhancer | acid | additive (chemical irritation) | | | | | | |
| Comparative example 1 | acrylic | HER2/neu E75 (10) | IMQ (3) | PEP (1) | IPM (34.4) | none | none | 52 | none | 10 | genetically modified | 78 | |
| Example 1 | acrylic | HER2/neu E75 (10) | IMQ (3) | PEP (1) | IPM (25.8) | myristic acid (8.6) | none | 98 | none | 10 | genetically modified | 117 | 30 |
| Example 2 | acrylic | HER2/neu E75 (10) | IMQ (3) | PEP (1) | IPM (25.8) | lauric acid (8.6) | none | | none | | genetically modified | | |
| Example 3 | acrylic | HER2/neu E75 (10) | IMQ (3) | PEP(1) | IPM (25.8) | isostearic acid (8.6) | none | | none | | genetically modified | | |
| Example 4 | acrylic | HER2/neu E75 (10) | IMQ (3) | PEP (1) | IPM (25.8) | palmitic acid (8.6) | none | | none | | genetically modified | | |
| Example 5 | acrylic | HER2/neu E75 (10) | IMQ (3) | PEP (1) | IPM (25.8) | oleic acid (8.6) | none | | none | | genetically modified | | |
| Comparative example 2 | acrylic | HER2/neu E75 (10) | IMQ (3) | PADRE (1) | IPM (34.4) | none | none | | none | 12 | genetically modified | 68 | |
| Example 6 | acrylic | HER2/neu E75 (10) | IMQ (3) | PADRE (1) | IPM (14.4) | lactic acid (20.0) | none | | none | 12 | genetically modified | 210 | |
| Comparative example 3 | saline | HER2/neu E75 (0.125) | Montanide ISA51VG (50) | | none | none | none | | none | | genetically modified | 110 | 28 |

The number in parentheses () is the blended proportion of each component (part by weight). (It has the same meaning in the following Tables.)
acrylic: acrylic adhesive
IMQ: Imiquimod
PEP: Peptide-25 (SEQ ID NO: 13)
PADRE: Universal helper peptide (SEQ ID NO: 15)

IPM: isopropyl myristate, manufactured by Croda Japan KK

Table 2

| | Composition | | | | | | | TSLP (pg/mg protein) | physical irritation | TEWL (g/h·$m^2$) | mouse for immunological evaluation | Results of imnumization (ELISPOT average spot number) |
|---|---|---|---|---|---|---|---|---|---|---|---|---|
| | base | antigen peptide | cellular immunity induction promoter | | skin permeability enhancer | acid | additive (chemical irritation) | | | | | |
| Comparative example 4 | acrylic | IPEP87 (10) | IMQ (3) | PEP (1) | IPM (35.2) | none | none | 50 | none | 10 | genetically modified | 28 |
| Example 7 | acrylic | IPEP87 (10) | IMQ (3) | PEP (1) | IPM (25.8) | myristic acid (8.6) | none | 95 | none | 10 | genetically modified | 132 |

Table 3

| | Composition | | | | | | | TSLP (pg/mg protein) | physical irritation | TEWL (g/h·m$^2$) | mouse for immmological evaluation | Results of immunization (ELISPOT average spot number) |
|---|---|---|---|---|---|---|---|---|---|---|---|---|
| | base | antigen peptide | cellular immunity induction promoter | | skin permeability enhancer | acid | additive (chemical irritation) | | | | | |
| Comparative example 5 | PIB | PR1 (10) | IMQ (3) | PEP (1) | IPM (35.2) | none | none | 26 | none | 10 | genetically modified | 14 |
| Example 8 | PIB | PR1 (10) | IMQ (3) | PEP (1) | IPM (25.8) | myristic acid (8.6) | none | 89 | none | 10 | genetically modified | 33 |
| Example 9 | acrylic | PR1 (10) | IMQ (3) | PEP (1) | IPM (25.8) | myristic acid (8.6) | none | 110 | none | 10 | genetically modified | 16 |
| PIB: PIB rubber adhesive | | | | | | | | | | | | |

Liquid Formulation for Sublingual Administration

Liquid formulations having compositions as shown in Tables 4, 5, and 6 were prepared to obtain samples for immunity induction study. Specifically, to an antigen (a peptide or a protein), a cellular immunity induction promoter and a pharmacologically acceptable acid in amounts as shown in Tables 4, 5, and 6 were added 20 parts by weight of an additive (DMSO) and a physiological saline solution as a base material so that the total amount became 100 parts by weight, and then blended to form a liquid formulation for sublingual administration.
Survivin-2B peptide (TFA salt) and Peptide-25B (Pep25B) (TFA salt) were prepared by chemical synthesis, and purified by HPLC before use. OVA protein was purchased from Sigma-Aldrich. The lipopolysaccharide derived from Pantoea was manufactured by Institute of applied technology for innate immunity. The other antigen peptides and the cellular immunity induction promoter are available from the same suppliers as described in tape preparations.

Immunity Induction Study 2 in Mouse (Sublingual Administration)

The liquid formulations for sublingual administration as prepared in the above were used to examine an immunity induction study. The test was performed by ELISPOT method. Specifically, for a single administration, a mouse was anesthetized, and then each liquid formulation for sublingual administration was administered at a sublingual region and then kept for 2 minutes. After that, the mouse was reared for 6 days. For two administrations, the procedure was repeated 6 days after the first administration. Six days after the second administration, the spleen of the mouse was removed. The level of cellular immunity induction specific for the antigen was evaluated by ELISPOT method as described later. The ELISPOT method was carried out in the same method as Immunity Induction Study in Mouse 1.
The results of the immunity induction study, as well as the mouse used, the amount of administration, and the number of administration are shown in Tables 4 and 5. In the Tables, the genetically modified mouse is a genetically modified mouse in which the cellular immunity induction by HLA-A*0201 type MHC-restricted peptide can be evaluated. In addition, for comparison, the result of the immunity study using injections as described later (Comparative Example 3) is shown at the bottom of the Table.

Table 4

| | composition | | | | | Dose | number of administra tions | mouse for immunological evaluation | Results of immunization (ELISPOT average spot number) |
|---|---|---|---|---|---|---|---|---|---|
| | base | antigen | cellular immunity induction promoter | | acid | | | | |
| Comparative example 6 | saline | survivin 2B (2.5) | LPS (0.1) | PEPB (0.3) | none | 20 µL | 2 | BALB/c | 49 |
| Example 10 | saline | survivin 2B (2.5) | LPS (0.1) | PEPB (0.3) | myristic acid (0.05) | 20 µL | 2 | BALB/c | 80 |
| Example 11 | saline | survivin 2B (2.5) | LPS (0.1) | PEPB (0.3) | isostearic acid(0.05) | 20 µL | 2 | BALB/c | 72 |
| Example 12 | saline | survivin 2B (2.5) | LPS (0.1) | PEPB (0.3) | lactic acid(0.05) | 20 µL | 2 | BALB/c | 76 |
| Example 13 | saline | survivin 2B (2.5) | LPS (0.1) | PEPB (0.3) | citric acid(0.05) | 20 µL | 2 | BALB/c | 70 |
| Example 14 | saline | survivin 2B (2.5) | LPS (0.1) | PEPB (0.3) | malic acid (0.05) | 20 µL | 2 | BALB/c | |
| Example 15 | saline | survivin 2B (2.5) | LPS (0.1) | PEPB (0.3) | salicylic acid (0.05) | 20 µL | 2 | BALB/c | |
| Example 16 | saline | survivin 2B (2.5) | LPS (0.1) | PEPB (0.3) | maleic acid (0.05) | 20 µL | 2 | BALB/c | 61 |

LPS: lipopolysaccharide derived from Pantoea
PEPB: Peptide-25B (SEQ ID NO: 14)

Table 5

| | composition | | | | | Dose | number of administra tions | mouse for immunological evaluation | Results of immunization (ELISPOT average spot number) |
|---|---|---|---|---|---|---|---|---|---|
| | base | antigen | cellular immunity induction promoter | | acid | | | | |
| Comparative example 7 | saline | HER2/neu E75 (1.25) | LPS (0.1) | PEP (0.3) | none | 20 μL | 2 | genetically modified | 30 |
| Example 17 | saline | HER2/neu E75 (1.25) | LPS (0.1) | PEP (0.3) | myristic acid (0.05) | 20 μL | 2 | genetically modified | 41 |
| Example 18 | saline | HER2/neu E75 (1.25) | LPS (0.1) | PEP (0.3) | isostearic acid (0.05) | 20 μL | 2 | genetically modified | 38 |
| Example 19 | saline | HER2/neu E75 (1.25) | LPS (0.1) | PEP (0.3) | lactic acid (0.05) | 20 μL | 2 | genetically modified | 37 |
| Example 20 | saline | HER2/neu E75 (1.25) | LPS (0.1) | PEP (0.3) | citric acid (0.05) | 20 μL | 2 | genetically modified | 38 |
| Comparative example 3 | saline | HER2/neu E75 (0.125) | Montanide ISA51VG (50) | | none | 200 μL | 1 | genetically modified | 110 |

Table 6

| | Composition | | | | | Dose | number of administra tions | mouse for immunological evaluation | Results of immunization (ELISPOT average spot number) |
|---|---|---|---|---|---|---|---|---|---|
| | base | antigen | cellular immunity induction promoter | | acid | | | | |
| Comparative example 8 | saline | OVA protein (1.25) | LPS (0.1) | none | none | 20 µL | 2 | BALB/c | 120 |
| Example 21 | saline | OVA protein (1.25) | LPS (0.1) | none | myristic acid (0.05) | 20 µL | 2 | BALB/c | 231 |
| Comparative example 9 | saline | OVA protein (1.25) | LPS (0.1) | PEPB (0.3) | none | 20 µL | 2 | BALB/c | 142 |
| Example 22 | saline | OVA protein (1.25) | LPS (0.1) | PEPB (0.3) | myristic acid (0.05) | 20 µL | 2 | BALB/c | 405 |
| Example 23 | saline | OVA protein (1.25) | LPS (0.1) | PEPB (0.3) | isostearic acid(0.05) | 20 µL | 2 | BALB/c | 380 |
| Example 24 | saline | OVA protein (1.25) | LPS (0.1) | PEPB (0.3) | lactic acid (0.05) | 20 µL | 2 | BALB/c | 365 |
| Example 25 | saline | OVA protein (1.25) | LPS (0.1) | PEPB (0.3) | citric acid (0.05) | 20 µL | 2 | BALB/c | 345 |

Subcutaneous Injection

Subcutaneous injections having compositions as shown in Table 7 were prepared to obtain samples for immunity induction study. Specifically, to an antigen peptide and Montanide ISA51VG (Freund Corporation) as an adjuvant in amounts as shown in Table 7 were added 0.5 parts by weight of an additive (DMSO) and a physiological saline solution as a base material so that the total amount became 100 parts by weight and then blended to form an injection. The antigen peptides used were all in the form of a TFA salt. The antigen peptides are available from the same suppliers as described in liquid formulations for sublingual administration.

Immunity Induction Study 3 in Mouse (Subcutaneous Injection)

The mouse immunity induction study was carried out with subcutaneous injections. The immunity induction level was evaluated by ELISPOT method. Specifically, 200 μL of an injection was administered subcutaneously at the back skin of a mouse. Then, the mouse was reared for 6 days. Six days after the administration, the spleen of the mouse was removed, and the level of the cellular immunity induction specific for the antigen was evaluated by the ELISPOT method. The number of administration was one. The ELISPOT method was carried out in the same method as Immunity Induction Study 1 in Mouse.
The results of the immunity induction study and the mouse used are shown in Table 7. In the Table, the genetically modified mouse is a genetically modified mouse in which the immunity induction by HLA-A*0201 type MHC-restricted peptide can be evaluated.

Table 7

| | composition | | | mouse for immunological evaluation | Results of immunization (ELISPOT average spot number) |
|---|---|---|---|---|---|
| | base | antigen peptide | cellular immunity induction promoter | | |
| Comparative example 3 | saline | HER2/neu E75 (0.125) | Montanide ISA51VG (50) | genetically modified | 110 |

Liquid Formulation for External Use

The liquid formulations for external use having compositions as shown in Table 8 were prepared. Specifically, HER2/neu E75 peptide (TFA salt) and a cellular immunity induction promoter in an amount as shown in Table 8, and 20 parts by weight of DMSO were blended to form a mixture, and added a base material to the mixture so that the total amount is 100 parts by weight, and then blended the mixture to form a liquid formulation for external use. The base material used was previously prepared by mixing and blending propylene glycol (PG) and oleyl alcohol (OA) in a weight ratio of 98 : 2. Imiquimod (IMQ) was purchased from Tokyo Chemical Industry Co., Ltd. The HER2/neu E75 peptide (TFA salt) and the Peptide-25 (TFA salt) was prepared by chemical synthesis, and purified by HPLC before use.
A cellulose non-woven fabric (area 0.8 $cm^2$) was attached to a middle part of an adhesive tape for fixing to form a composite substrate. The non-woven fabric part of the composite substrate was immersed in 67 μL of the liquid formulation for external use thus prepared to form a sample for immunity induction study.

Immunity Induction Study 4 in Mouse (Liquid Formulation for External Use)

The liquid formulations for external use as prepared in the previous paragraph were used to examine an immunity. induction study in mouse using model animals for immunological evaluation. The immunity induction level was evaluated by ELISPOT method. Specifically, the fur of the back skin of a mouse was cut, and the mouse was kept in a rearing period for curing of the skin damage. After the period, each sample was administered to the back skin of the mouse for a predetermined period, and then removed. After a predetermined day, the level of the cellular immunity induction specific for the antigen was evaluated. On a predetermined day after the administration, the spleen of the mouse was extracted to prepare a suspension of the spleen cells. Spleen cells ($3\times10^6$ cells/well) and an antigen peptide (100 μM) were added with a culture solution in a well of ELISPOT plate containing a fixed anti-mouse IFN-γ antigen, and co-cultured at 37°C in 5% $CO_2$ for 20 hours. The number of the spot which IFN-γ was produced was evaluated by ELISPOT method (the number of the spot/$3\times10^6$ cells). The dosage of the liquid formulation for external use was all 67 μL as described above, and the number of the administration was one (24hr/week), the extraction of the spleen was 6 days after the administration.

For some liquid formulations for external use, according to the same method as described in the above Immunity Induction Study 1 in Mouse, cutaneous TSLP level in the skin of the mouse after administration and transepidermal water loss of the mouse before administration were also measured. The mouse used for measuring TSLP level and transepidermal water loss is C57BL/6 mouse for model animals for evaluating skin irritation.

The results of the immunity induction study, and the measurement results of TSLP level and transepidermal water loss are shown in Table 8. The mouse used is a mouse in which the cellular immunity induction by HLA-A*0201 type MHC-restricted peptide can be evaluated. In addition, for the comparison, the result of the immunity study using injections (Reference Example 1) is shown at the bottom of the Table.

Table 8

| | composition | | | | | chemical irritation | TSLP (pg/mg protein) | physical irritation | TEWL (g/h·m$^2$) | Results of immunization (ELISPOT average spot number) |
|---|---|---|---|---|---|---|---|---|---|---|
| | base | antigen peptide | cellular immunity induction promoter | | acid | | | | | |
| Comparative example 10 | PG/OA [98/2] | HER2/neu E75 (10) | none | none | none | none | | none | 10 | 5 |
| Comparative example 11 | PG/OA [98/2] | HER2/neu E75 (10) | IMQ (3) | PEP (0.3) | none | none | 224 | none | 10 | 882 |
| Example 26 | PG/OA [98/2] | HER2/neu E75 (10) | IMQ (3) | PEP (0.3) | lactic acid (1) | none | | none | 10 | 1150 |
| Example 27 | PG/OA [98/2] | HER2/neu E75 (10) | IMQ (3) | PEP (0.3) | methanesulf onic acid (1) | none | | none | 10 | 967 |
| Example 28 | PG/OA [98/2] | HER2/neu E75 (10) | IMQ (3) | PEP (0.3) | D,L-malic acid (1) | none | | none | 10 | 933 |
| Example 29 | PG/OA [98/2] | HER2/neu E75 (10) | IMQ (3) | PEP (0.3) | maleic acid (1) | none | | none | 10 | 925 |
| Comparative example 3 | saline | HER2/neu E75 (0.125) | Montanide ISA51VG (50) | | none | none | | none | | 110 |

PG/OA: mixture of propylene glycol and oleyl alcohol (both are manufactured by Wako Pure Chemical Industries, Ltd.). The number in square brackets [] shows the ratio of PG to OA.
The number in parentheses () is the amount of each component (parts by weight). (It has the same meaning in the following Tables.)
IMQ: Imiquimod
PEP: Peptide-25

Cream Formulation

Cream formulation having compositions as shown in Table 10 were prepared. Specifically, HER2/neu E75 peptide (TFA salt) and a cellular immunity induction promoter in an amount as shown in Table 10, 15 parts by weight of DMSO, and optionally an additive were blended to form a mixture, and added a base material (acid-free cream) to the mixture so that the total amount is 100 parts by weight, and then blended the mixture to form a cream formulation. The acid-free cream used was prepared by mixing and blending its components in the composition as shown in Table 9.

A PET film/PET non-woven fabric laminate (area 0.7 $cm^2$) was attached to a middle part of an adhesive tape for fixing so that the PET film side of the laminate is contacted with the adhesive tape to form a composite substrate. Four mg of the cream formulation was applied on the non-woven fabric part of the composite substrate to form a sample for immunity induction study.

HER2/neu E75 peptide (TFA salt) and the cellular immunity induction promoter were available from the same supplier as described in the above tape preparations and the liquid formulations for external use. PADRE (acetate) was chemically synthesized and purified by HPLC, which was used.

Immunity Induction Study 5 in Mouse (Cream Formulation)

The cream formulations as prepared in the previous paragraph were used to examine an immunity induction study according to the same method as the above Immunity Induction Study 4. The number of the administration was performed once (24hr/week), and the spleen was extracted 6 days after the administration.

According to the same method as described in the above Immunity Induction Study 1, cutaneous TSLP level in the skin of the mouse after administration and transepidermal water loss of the mouse before administration were also measured. The mouse used for measuring TSLP level and transepidermal water loss is C57BL/6 mouse for model animals for evaluating skin irritation.

The results of the immunity induction study, and the measurement results of TSLP level and transepidermal water loss are shown in Table 10. The mouse used is a genetically modified mouse in which the cellular immunity induction by HLA-A*0201 type MHC-restricted peptide can be evaluated. In addition, for the comparison, the result of the immunity study using injections (Reference Example 1) is shown at the bottom of the Table.

Table 9

| | acid-free cream |
|---|---|
| white vaseline | 69.0 wt% |
| sorbitan monostearate | 0.8 wt% |
| benzyl alcohol | 2.7 wt% |
| cetanol | 2.7 wt% |
| stearyl alcohol | 4.0 wt% |
| polysorbate 60 | 4.0 wt% |
| concentrated glycerin | 2.7 wt% |
| water | 14.1 wt% |

White vaseline, sorbitan monostearate, benzyl alcohol, stearyl alcohol, polysorbate 60, and concentrated glycerin were purchased from Wako Pure Chemical Industries, Ltd. Cetanol was purchased from Tokyo Chemical Industry Co., Ltd.

Table 10

| | composition | | | | | | TSLP (pg/mg protein) | physical irritation | TEWL (g/h·m$^2$) | Results of immunization (ELISPOT average spot number) |
|---|---|---|---|---|---|---|---|---|---|---|
| | base | antigen peptide | cellular immunity induction promoter | | acid | additive (chemical irritation) | | | | |
| Comparative example 12 | acid-free cream | HER2/neu E75 (5) | IMQ (3) | PADRE (1) | none | none | | none | 12 | 45 |
| Example 30 | acid-free cream | HER2/neu E75 (5) | IMQ (3) | PADRE (1) | myristic acid (5) | none | | none | 12 | 106 |
| Example 31 | acid-free cream | HER2/neu E75 (5) | IMQ (3) | PADRE (1) | isostearic acid (5) | none | | none | 12 | 65 |
| Example 32 | acid-free cream | HER2/neu E75 (5) | IMQ (3) | PADRE (1) | lauric acid (5) | none | | none | 12 | 110 |
| Example 33 | acid-free cream | HER2/neu E75 (5) | IMQ (3) | PADRE (1) | decanoic acid (5) | none | | none | 12 | 123 |
| Example 34 | acid-free cream | HER2/neu E75 (5) | IMQ (3) | PADRE (1) | oleic acid (5) | none | | none | 12 | 97 |
| Example 35 | acid-free cream | HER2/neu E75 (5) | IMQ (3) | PADRE (1) | palmitic acid (5) | none | | none | 12 | 143 |
| Example 36 | acid-free cream | HER2/neu E75 (5) | IMQ (3) | PADRE (1) | stearic acid (5) | none | | none | 12 | 140 |
| Example 37 | acid-free cream | HER2/neu E75 (5) | IMQ (3) | PADRE (1) | lactic acid (5) | none | | none | 12 | 150 |
| Example 38 | acid-free cream | HER2/neu E75 (5) | IMQ (3) | PADRE (1) | methanesulfonic acid (5) | none | | none | 12 | 138 |
| Example 39 | acid-free cream | HER2/neu E75 (5) | IMQ (3) | PADRE (1) | malic acid (5) | none | | none | 12 | 82 |
| Example 40 | acid-free cream | HER2/neu E75 (5) | IMQ (3) | PADRE (1) | salicylic acid (5) | none | | none | 12 | 132 |
| Comparative example 3 | saline | HER2/neu E75 (0.125) | Montanide ISA51VG (50) | | | none | | none | | 110 |

As shown in Tables 1, 2, 3, 8 and 10, it was confirmed that myristic acid, lauric acid, isostearic acid, decanoic acid, palmitic acid, oleic acid, stearic acid, lactic acid, methanesulfonic acid, malic acid, maleic acid and salicylic acid were suitable for the promotion of immunity induction via transdermal immunization. Among them, myristic acid, isostearic acid, decanoic acid, lauric acid, oleic acid, palmitic acid, stearic acid, lactic acid, methanesulfonic acid, malic acid, maleic acid and salicylic acid were preferable, and further, myristic acid, lauric acid, decanoic acid, palmitic acid, stearic acid, lactic acid, salicylic acid and methanesulfonic acid were particularly preferable.

As shown in Tables 4, 5 and 6, it was confirmed that myristic acid, isostearic acid, lactic acid, citric acid, malic acid, salicylic acid and maleic acid were suitable for the promotion of immunity induction using a mucosal vaccine. Among them, myristic acid, isostearic acid, lactic acid, citric acid and maleic acid were preferable, and further, myristic acid, isostearic acid, lactic acid and citric acid were particularly preferable.

*In vivo* CTL assay confirmed that the tape preparation of Example 1 exerts an effect equivalent to or stronger than that of the immunization with an injection.

Based on the above described results, it was confirmed that a vaccine composition for transdermal or transmucosal administration intended for the induction of cellular immunity comprising (i) an antigen and (ii) a pharmacologically acceptable acid or a pharmacologically acceptable salt thereof as a first cellular immunity induction promoter is useful.

## Claims

1. A vaccine composition for transdermal or transmucosal administration for use in the induction of cellular immunity, wherein the vaccine comprises:

   (i) an antigen; and

(ii) a pharmacologically acceptable acid or a pharmacologically acceptable salt thereof as a first cellular immunity induction promoter.

**2.** The vaccine composition according to claim 1, wherein the composition is for use in the treatment of a cancer.

**3.** The vaccine composition according to claim 1, wherein the composition is for use in the treatment of a viral disease.

**4.** The vaccine composition according to any one of claims 1-3, wherein the pharmacologically acceptable acid or the pharmacologically acceptable salt thereof is an organic acid or a pharmacologically acceptable salt thereof.

**5.** The vaccine composition according to claim 4, wherein the organic acid or the pharmacologically acceptable salt thereof is an organic compound having a carboxyl group or an organic compound having a sulfo group, or a pharmacologically acceptable salt thereof.

**6.** The vaccine composition according to claim 4, wherein the organic acid or the pharmacologically acceptable salt thereof is a saturated or unsaturated, linear or branched fatty acid with a saturated linear moiety having a carbon number of 8 to 20, lactic acid, malic acid, salicylic acid, maleic acid, citric acid, an organic compound having a sulfo group, or a pharmacologically acceptable salt thereof.

**7.** The vaccine composition according to claim 4, wherein the organic acid or the pharmacologically acceptable salt thereof is a fatty acid selected from the group consisting of decanoic acid, lauric acid, myristic acid, isostearic acid, palmitic acid, stearic acid and oleic acid, or lactic acid, salicylic acid, citric acid, methanesulfonic acid, or a pharmacologically acceptable salt thereof.

**8.** The vaccine composition according to any one of claims 1-7, further comprising at least one second cellular immunity induction promoter selected from the group consisting of a TLR ligand, a cyclic dinucleotide, a helper peptide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor. agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-$\beta$ production inhibitor, and a Th2 cytokine inhibitor.

**9.** The vaccine composition according to claim 8, wherein the second cellular immunity induction promoter is a helper peptide.

**10.** The vaccine composition according to claim 8, wherein the second cellular immunity induction promoter is a combination of a helper peptide and at least one substance selected from the group consisting of a TLR ligand, a cyclic dinucleotide, an immunomodulatory small molecule drug, a cyclooxygenase inhibitor, a prostaglandin receptor antagonist, a prostaglandin receptor agonist, a TSLP production inhibitor, an adenylate cyclase inhibitor, an omega-3 fatty acid, a PPAR agonist, a dopamine receptor antagonist, a dopamine receptor agonist, a histamine receptor agonist, a histamine receptor antagonist, a serotonin receptor agonist, a serotonin receptor antagonist, a vasopressin receptor antagonist, a vasopressin receptor agonist, a muscarine receptor antagonist, a muscarine receptor agonist, an adrenergic receptor antagonist, an adrenergic receptor agonist, an angiotensin receptor agonist, a GABA receptor agonist, a thrombin receptor antagonist, a thrombin receptor agonist, an opioid receptor agonist, an ADP receptor agonist, a leukotriene receptor antagonist, a leukotriene receptor agonist, a melatonin receptor agonist, a somatostatin receptor agonist, a cannabinoid receptor agonist, a sphingosine-1 phosphate receptor agonist, a metabotropic glutamate receptor agonist, a phospholipase A2 inhibitor, a TGF-$\beta$ production inhibitor and a Th2 cytokine inhibitor.

**11.** The vaccine composition according to any one of claims 1-10, wherein the antigen is a peptide selected from the group consisting of survivin-2B peptide and/or a modified survivin-2B peptide, GPC3 peptide and/or a modified GPC3 peptide, HER2/neu_A24 peptide and/or a modified HER2/neu_A24 peptide, MAGE3_A24 peptide and/or a modified MAGE3_A24 peptide, IPEP87 peptide and/or a modified IPEP87 peptide, PR1 peptide and/or a modified PR1 peptide, HER2/neu_A02 peptide and/or a modified HER2/neu_A02 peptide, MAGE3_A02 peptide and/or a

modified MAGE3_A02 peptide, HBVenv peptide and/or a modified HBVenv peptide, and MUC1 peptide and/or a modified MUC1 peptide.

**12.** The vaccine composition for transdermal administration according to any one of claims 1-11, wherein the composition is administered under a mildly irritating condition.

**13.** The vaccine composition for transdermal administration according to claim 12, wherein the mildly irritating condition is a condition under which transepidermal water loss (TEWL) in a model animal for skin irritation evaluation before the administration of the composition is 50 $g/h \cdot m^2$ or less.

**14.** The vaccine composition for transdermal administration according to claim 12 or 13, wherein the mildly irritating condition is a condition under which the cutaneous TSLP level in a model animal for skin irritation evaluation at completion of the administration of the composition is 10000 pg/mg protein or less.

Europäisches Patentamt
European Patent Office
Office européen des brevets

# EUROPEAN SEARCH REPORT

Application Number
EP 14 00 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/026111 A1 (US HEALTH [US]; ZHU QING [US]; BERZOFSKY JAY A [US]; TALTON JAMES [US]) 3 March 2011 (2011-03-03)<br>* page 1 *<br>* page 42 - page 45 *<br>* examples 1-7; table 2 * | 1-11 | INV.<br>A61K39/00<br>A61K39/12 |
| X | EP 1 384 403 A1 (US GOV SEC ARMY [US]) 28 January 2004 (2004-01-28)<br>* examples 21,26 *<br>* paragraph [0055] *<br>* paragraph [0113] * | 1-14 | |
| X | WO 00/61184 A2 (GLENN GREGORY M [US]; SCHARTON KERSTEN TANYA [US]) 19 October 2000 (2000-10-19)<br>* example 4 *<br>* page 9, last paragraph * | 1-14 | |
| X | HOSOI AKIHIRO ET AL: "Memory Th1 cells augment tumor-specific CTL following transcutaneous peptide immunization", CANCER RESEARCH, vol. 68, no. 10, May 2008 (2008-05), pages 3941-3949, XP055109896, ISSN: 0008-5472<br>* page 3941 - page 3942, paragraph top *<br>* page 3944; figure 3 * | 1-14 | TECHNICAL FIELDS SEARCHED (IPC)<br>A61K |

The present search report has been drawn up for all claims

1

| Place of search | Date of completion of the search | Examiner |
|---|---|---|
| The Hague | 23 May 2014 | Mata Vicente, Teresa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 00 0322

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2014

| Patent document cited in search report | Publication date | Patent family member(s) | Publication date |
|---|---|---|---|
| WO 2011026111 A1 | 03-03-2011 | NONE | |
| EP 1384403 A1 | 28-01-2004 | AT 274805 T | 15-09-2004 |
| | | AT 349890 T | 15-01-2007 |
| | | AU 744537 B2 | 28-02-2002 |
| | | BR 9712952 A | 07-12-1999 |
| | | CA 2272417 A1 | 22-05-1998 |
| | | CN 1241906 A | 19-01-2000 |
| | | DE 69730534 D1 | 07-10-2004 |
| | | DE 69730534 T2 | 15-09-2005 |
| | | DE 69737218 T2 | 08-11-2007 |
| | | EP 1014787 A1 | 05-07-2000 |
| | | EP 1384403 A1 | 28-01-2004 |
| | | EP 1557177 A1 | 27-07-2005 |
| | | EP 2272526 A2 | 12-01-2011 |
| | | IL 129919 A | 13-04-2008 |
| | | JP 4584361 B2 | 17-11-2010 |
| | | JP 2001511115 A | 07-08-2001 |
| | | KR 20000053306 A | 25-08-2000 |
| | | NZ 335749 A | 26-01-2001 |
| | | US 5980898 A | 09-11-1999 |
| | | US 7037499 B1 | 02-05-2006 |
| | | US 2006269593 A1 | 30-11-2006 |
| | | WO 9820734 A1 | 22-05-1998 |
| WO 0061184 A2 | 19-10-2000 | AT 529130 T | 15-11-2011 |
| | | AU 4188100 A | 14-11-2000 |
| | | CA 2368655 A1 | 19-10-2000 |
| | | EP 1165132 A2 | 02-01-2002 |
| | | EP 2368575 A2 | 28-09-2011 |
| | | ES 2372592 T3 | 24-01-2012 |
| | | JP 4932086 B2 | 16-05-2012 |
| | | JP 2003523937 A | 12-08-2003 |
| | | US 2004028727 A1 | 12-02-2004 |
| | | US 2005287157 A1 | 29-12-2005 |
| | | WO 0061184 A2 | 19-10-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080193487 A
- JP 2002531415 A
- US 20080112974 A
- JP H07505883 A
- JP 2007529531 A
- WO 4422903 A
- WO 200006602 A
- WO 2005095598 A
- WO 2007097358 A
- WO 2008081701 A
- WO 9850399 A
- US 6303347 B
- US 6764840 B


### Non-patent literature cited in the description


- **HOSOI AKIHIRO et al.** *Cancer Research,* 2008, vol. 68, 3941-3949
- **ZHENGRONG CUI et al.** *Pharmaceutical Research,* 2002, vol. 19 (7), 947-953
- **KAWAI et al.** *Nucleic Acids Research,* vol. 3, 103-4